# EUROPEAN PATENT APPLICATION

(11) **EP 1 770 089 A1**
(43) Date of publication of application: **04.04.2007**
(21) Application number: 05292048.5
(22) Date of filing: 03.10.2005
(51) Int. Cl.: C07D 307/91, C07D 493/04, C07D 493/14, A61K 31/352, A61K 31/357, A61P 31/04, A61K 31/343

(54) **Pyranodibenzofuran derivatives with antifungal and antibacterial activity**

(71) Applicant: INSTITUT PASTEUR, 75015 Paris (FR); CENTRE NATIONAL DE LA RECHERCHE SCIENTIFIQUE (CNRS), 75016 Paris (FR); Université Rene Descartes, 75006 Paris (FR)
(72) Inventor: Brodin, Priscille Marie Monique, 75014 Paris (FR); Prado, Soizic, 75005 Paris (FR); Cole, Stewart T., 92140 Clamart (FR); Koch, Michel, 78170 La Celle Saint Cloud (FR); Tillequin, Fran[ois, 75014 Paris (FR); Michel, Sylvie, 75002 Paris (FR)
(74) Representative: Warcoin, Jacques

(57) **Abstract**

Compound of the general formula : 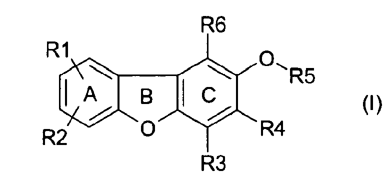 and in particular compounds of formula 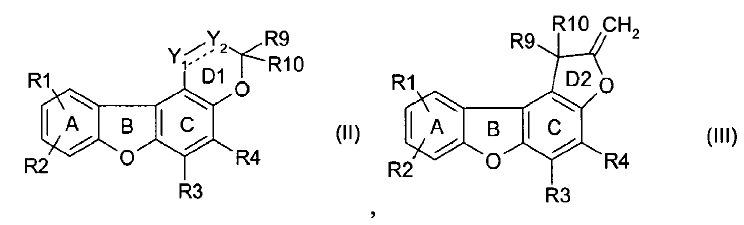 or 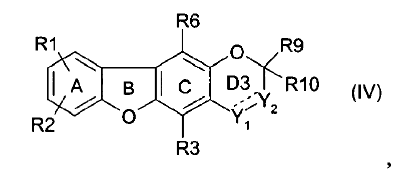 their process of preparation and their use as medicament for the treatment of fungal or bacterial infections, in particular mycobacterial infection.

## Description

The present invention concerns new pyranodibenzofuran compounds with antifungal and antibacterial, particularly anti-mycobacterial, activity, as well as methods to synthesize such compounds. The new compounds, or pharmaceutical compositions comprising them, may be used as medicaments for the treatment of mycobacterial infections, in particular tuberculosis infection, including infections by virulent and/or drug resistant *Mycobacterium tuberculosis* strains. These compounds might be particularly useful for immunodeficient patients who may reactivate a latent tuberculosis infection.

Tuberculosis (TB) remains a leading infectious disease accounting for more than 2 million deaths and 8 million new cases each year in the world. Though widely prescribed, the live vaccine BCG fails to prevent pulmonary tuberculosis in adults and to confer any protection in African countries and India partly due to environmental mycobacterial interference. The human immunodeficiency virus pandemic and the decline of the healthcare systems also contribute to this increase in tuberculosis incidence in less-developed countries and in the former Soviet Union. The current short course chemotherapy (DOTS), which was established more than 30 years ago, consists of a combination of isoniazid (INH), rifampin (RIF), pyrazinamide and ethambutol or streptomycin. Its outcome strongly depends on following the drug regimen for 6 months or more. Together with the resurgence of the disease, inappropriate regimens or inadequate compliance to DOTS further entail the rise of multiple drug resistant strains of tuberculosis (MDR-TB). When not restrained, MDR-TB spreads in the population with the same trend as the more sensitive strains. Thus, there is a pressing need for the development of a new class of antimycobacterial agents in order to fight the resistance and shorten the duration of therapy.

Apart from a nitroimidazopyran derivative, (PA-824), which gave promising results in preclinical trials (Stover, C. K. et al. Nature 2000, 405, 962-965), no new specific anti-TB agents have been introduced lately. Since natural products are a rich source of drug leads, attempts to identify a novel class of anti TB-agents in marine and plant extracts have been undertaken in the past (Okunade, A. L. et al. Phytochemistry 2004, 65, 1017-1032). New families of natural compounds exhibiting various antibacterial activities *in vitro* were thus reported (Copp, B. R. Nat. Prod.Rep. 2003, 20, 535-537). For instance, natural products bearing dimethylbenzopyran moiety 1 were isolated from Asteraceae, Rutaceae and Piperaceae (Ingolfsdottir, K. et al., Eur J Pharm Sci 1998, 6, (2), 141-4; Ingolfsdottir, K. Phytochemistry 2002, 61, 729-736; Muller, K. Appl. Microbiol. Biotechnol. 2001, 56, 9-16; Cocchietto, M. et al., Naturwissenschaften 2002, 89,137-146).

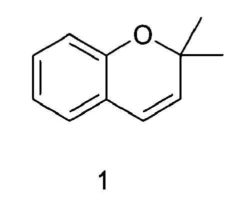

In addition, phytoalexins, which were found mainly in lichens and in fungus-infected Rosaceae and Liliaceae, quite often, harbor the dibenzofuran structure **2.** Both dimethylbenzopyran and dibenzofuran derived compounds have been reported to be of medicinal interest with potent antibacterial properties (Muller, K. Appl. Microbiol. Biotechnol. 2001, 56, 9-16; Cocchietto, M. et al. Naturwissenschaften 2002, 89, 137-146; Ingolfsdottir, K. Phytochemistry 2002, 61, 729-736).

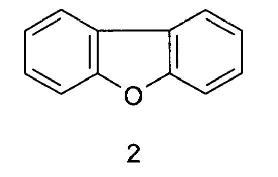

However, very few natural or synthetic compounds featuring the dimethylbenzopyran component as well as a dibenzofuran ring system have been described for the moment. Compounds in which one or both of the methyl groups of the dimethylbenzopyran component are replaced by a phenyl group have been described for their photochromic properties, but no biological activity of these compounds has been tested (Pozzo, J.L. et al. Can. J. Chem. 1996 74, 1649-1659). Compounds of the carbazole family in which the O atom of the furan ring is replaced by a N atom have also been described and shown to display antifungal and/or antibacterial activity, but the antifungal activity of compounds with a dimethylpyran ring was lower than that of compounds without the dimethylpyran ring (Pacher, T. et al. Phytochemistry 1999, 58, 129-135).

The inventors synthesized new compounds featuring a dimethylbenzopyran component as well as a dibenzofuran ring system and, contrary to carbazole family compounds, surprisingly found that these compounds displayed antifungal activity and high antimycobacterial activity.

The invention thus concerns a compound of the general formula : 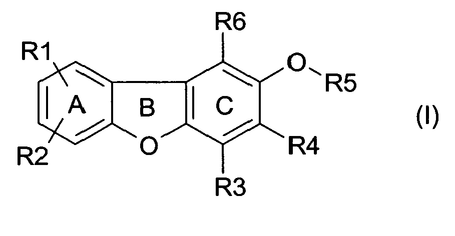 wherein :
- R1, R2 and R3 independently represent an hydrogen atom, a halogen atom , in particular F, Cl, or Br, a (C₁-C₆)alkyl, in particular methyl or ethyl, CF₃, NO₂, NH₂, NR7, NHCOR8, OH, OR8, CN, O(C₁-C₆alkyl)NR7, (C₁-C₆alkyl) NR7, or NR8 (C₁-C₆ alkyl)NR7, wherein NR7 represents a nitrogenous heterocycle, in particular 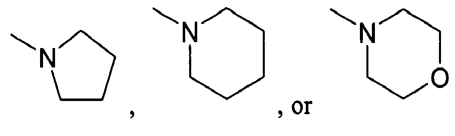 and R8 represents an hydrogen atom, or a (C₁-C₆)alkyl.
- R4 and R6 represent an hydrogen atom, a halogen atom , in particular F, Cl, or Br, a (C₁-C₆)alkyl, in particular methyl or ethyl, CF₃, NO₂, NH₂, NR7, NHCOR8, OH, OR8, CN, O(C₁-C₆ alkyl)NR7, (C₁-C₆ alkyl)NR7, or NR8(C₁-C₆ alkyl)NR7, wherein R7 and R8 are as defined above, and R5 represents 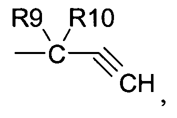 wherein R9 and R10 independently represent an hydrogen atom or a (C₁-C₆)alkyl, in particular methyl or ethyl.
- or R4 represents an hydrogen atom, a halogen atom , in particular F, Cl, or Br, a (C₁-C₆)alkyl, in particular methyl or ethyl, CF₃, NO₂, NH₂, NR7, NHCOR8, OH, OR8, CN, O(C₁-C₆ alkyl)NR7, (C₁-C₆ alkyl)NR7, or NR8(C₁-C₆ alkyl)NR7, wherein R7 and R8 are as defined above, and R5 and R6 taken together form a pyran ring D1, according to the following formula: 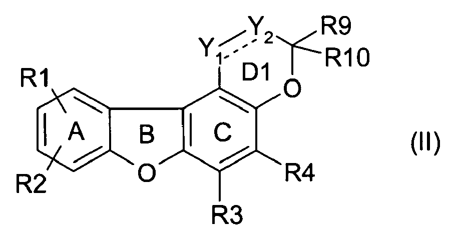 wherein R9 and R10 are as defined above,
   and wherein 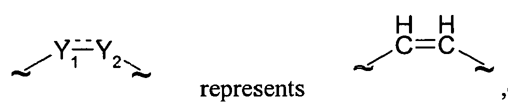 or 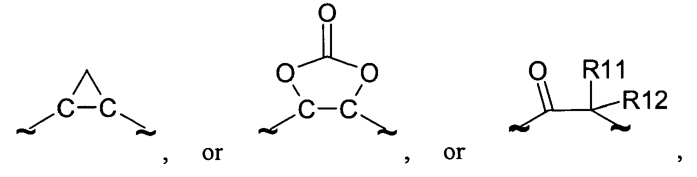 or 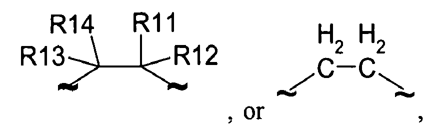 wherein R11, R12, R13 and R14 independently of each other represent an hydrogen atom, OH, O-C-O-(C₁-C₆)alkyl, or a (C₁-C₆)alkylcarbonyl(C₁-C₆)alkyl,
   or R5 and R6 taken together form a oxacyclopentan ring D2 according to the following formula : 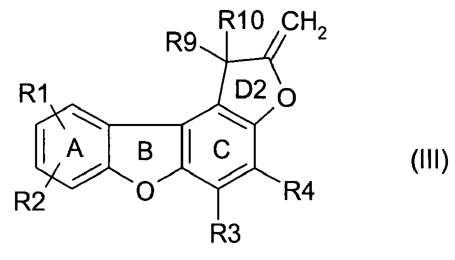 wherein R9 an R10 are as defined above,
- or R6 represents an hydrogen atom, a halogen atom , in particular F, Cl, or Br, a C₁-C₆ alkyl, in particular methyl or ethyl, CF₃, NO₂, NH₂, NR7, NHCOR8, OH, OR8, CN, O(C₁-C₆ alkyl)NR7, (C₁-C₆ alkyl)NR7, or NR8(C₁-C₆ alkyl)NR7, wherein R7 and R8 are as defined above, and R4 and R5 taken together form a pyran ring D3, according to the following formula: 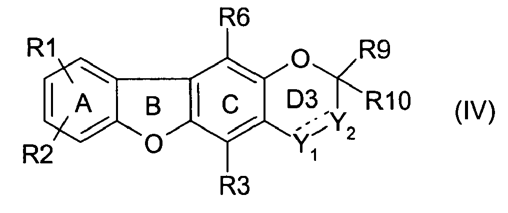 wherein R9, R10 and 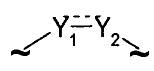 are as defined above,
   or a pharmaceutically acceptable salt, an isomer, an enantiomer, or a diastereoisomer thereof, or a mixture thereof.

For the purpose of the present invention, the term "(C₁-C₆)alkyl" is intended to mean any linear or branched saturated hydrocarbon radical having from one to six carbon atoms. Examples of (C₁-C₆)alkyl groups include a methyl or an ethyl group.

For the purpose of the present invention, the term "(C₁-C₆)alkylcarbonyl(C₁-C₆)alkyl" is intended to mean 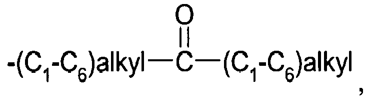 wherein (C₁-C₆)alkyl is as defined above. Examples of (C₁-C₆)alkylcarbonyl(C₁-C₆)alkyl groups include -CH₂-CO-CH₃, -CH₂-CO-CH₂-CH₃,- CH₂-CH₃-CO-CH₃,- CH₂-CH₃-CO-CH₂-CH₃.

As used herein, a "pharmaceutically acceptable salt" includes both acid and base addition salts. "Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid and the like, and organic acids such as acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. "Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminium salts and the like. Salts derived from organic bases include, but are not limited to, salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like.

As used herein, "isomer" compounds are compounds that have identical molecular formulae but that differ in the nature or the sequence of bonding of their atoms or in the arrangement of their atoms in space. Isomers that differ in the arrangement of their atoms in space are termed "stereoisomers". Stereoisomers that are not mirror images of one another are termed "diastereoisomers", and stereoisomers that are non-superimposable mirror images are termed "enantiomers", or sometimes optical isomers.

In an embodiment, a compound according to the invention, including a pharmaceutically acceptable salt, an isomer, an enantiomer, or a diastereoisomer thereof, or a mixture thereof, has the following general formula: 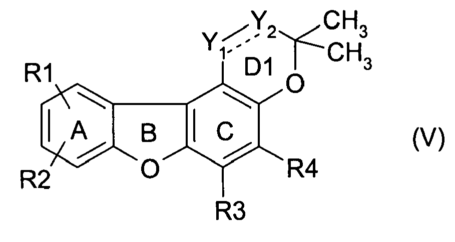 wherein R1, R2, R3, R4, and 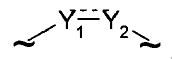 are as defined above.

In another embodiment, a compound according to the invention, including a pharmaceutically acceptable salt, an isomer, an enantiomer, or a diastereoisomer thereof, or a mixture thereof, has the following general formula: 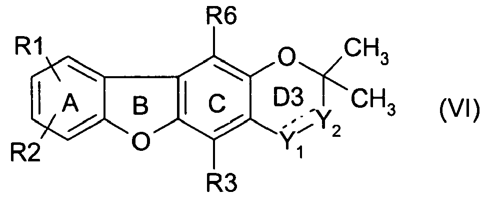 wherein R1, R2, R3, R6, and 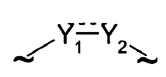 are as defined above.

In a preferred embodiment of a compound according to the invention, including a pharmaceutically acceptable salt, an isomer, an enantiomer, or a diastereoisomer thereof, or a mixture thereof, 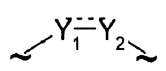 in ring D1 or D3 of general formulas (V) and (VI) represents

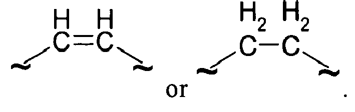

Advantageously, in a compound according to the invention, R1, R2, R3, R4 and R6 when they are not members of a pyran or oxacyclopentan ring, independently represent a hydrogen atom, a halogen atom, in particular F, Cl, or Br, a (C₁-C₆)alkyl, in particular methyl or ethyl, OH, or OR8, wherein R8 is as defined above.

More advantageously, in a compound according to the invention, R1, R2, R3, R4 and R6 when they are not members of a pyran or oxacyclopentan ring, are a hydrogen atom.

Preferred compounds according to the invention have one of the following formulas:

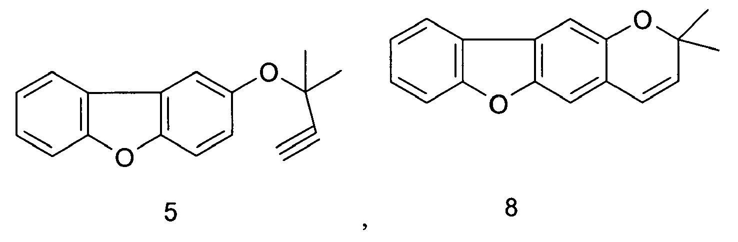

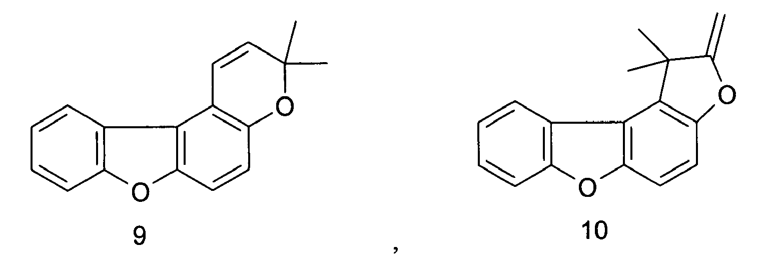

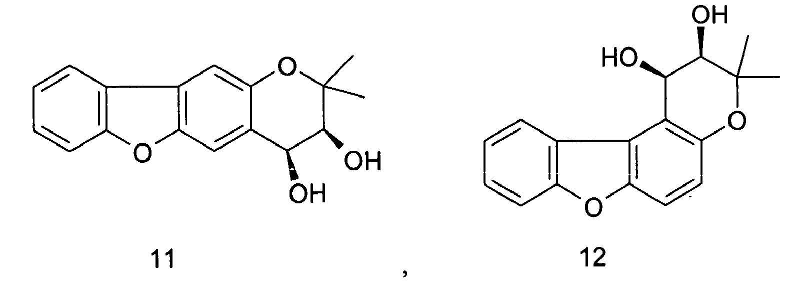

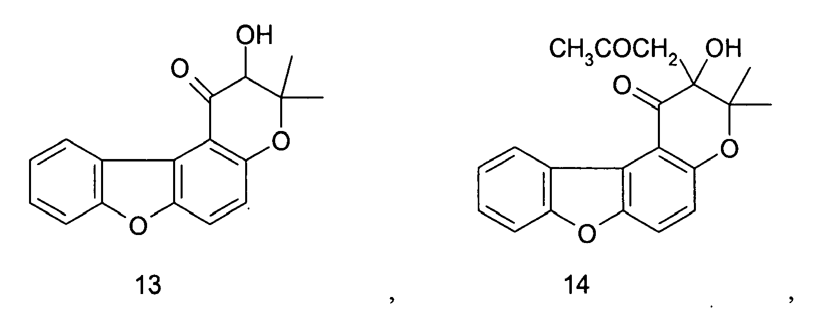

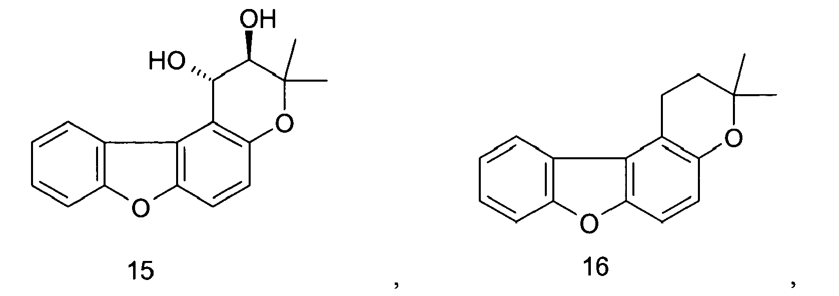

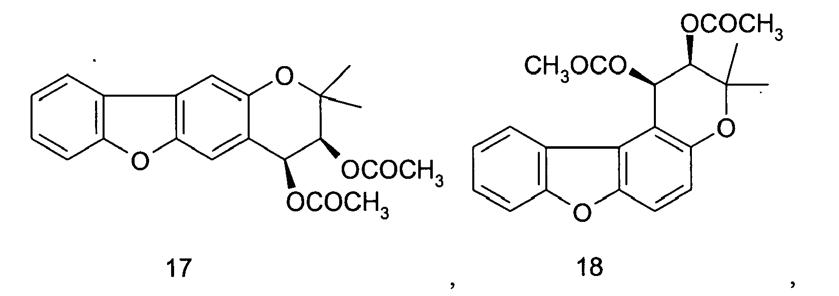

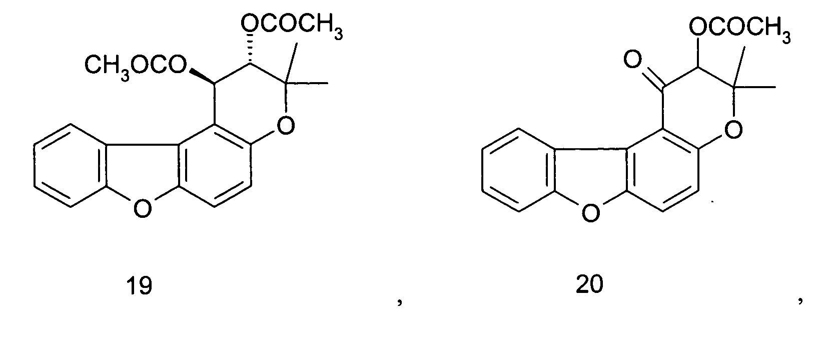

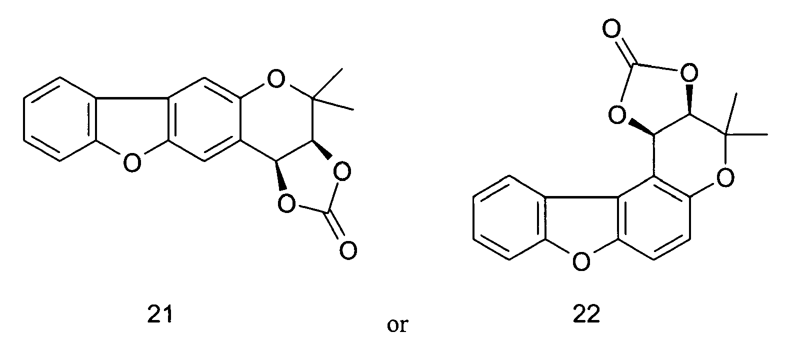

Most preferably, a compound according to the invention may have one of the following formulas:

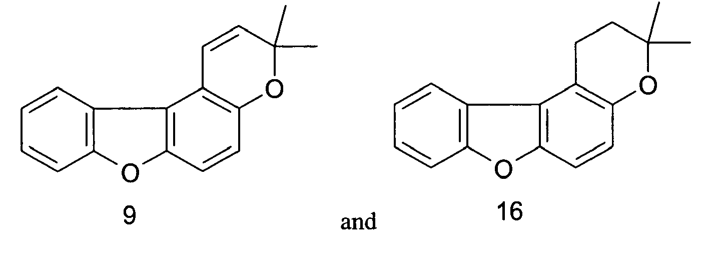

The invention also concerns a method for preparing a compound having the following general formula: 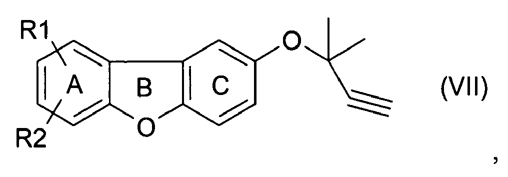 wherein R1 and R2 are as defined above, comprising:
(a) adding 1,8-Diazabicyclo[5.4.0]undec-7-ene (DBU) and copper chloride dihydrate to a cooled solution in anhydrous acetonitrile under inert atmosphere of a compound having the following formula 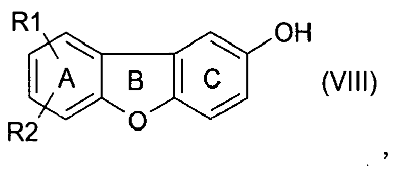 and then adding chlorobutyne,
(b) stirring at 0°C, advantageously between 2 and 48 hours, more advantageously for 5 hours,
(c) concentrating the mixture at reduced pressure
(d) partitioning the organic fraction and the aqueous fraction,
(e) washing the organic fraction successively with hydrochloric acid, sodium hydroxide, sodium hydrogenocarbonate and brine,
(f) removing the solvent to give compound of formula (VII).

The invention further concerns a method for preparing a compound having the following general formula: 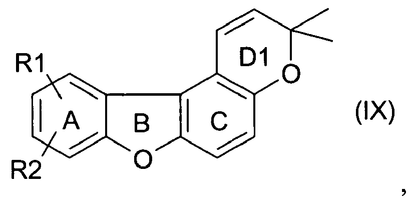 wherein R1 and R2 are as defined above, comprising:
(g1) heating the compound of formula 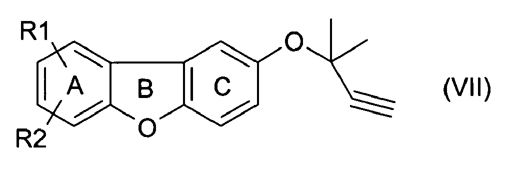 in a boiling solvent, advantageously chosen from DMF, N,N-dimethylaniline, N,N-diéthylaniline and 1,3-dichlorobenzene, more advantageously in 1,3-dichlorobenzene, advantageously between 3 and 24 hours, more advantageously during 12 hours,
(h1) purifying compound of formula (IX) by chromatography over silica gel and
(i1) recrystallizing the compound of formula (IX) in methanol.

Also included in the invention is a method for preparing a compound having the following general formula: 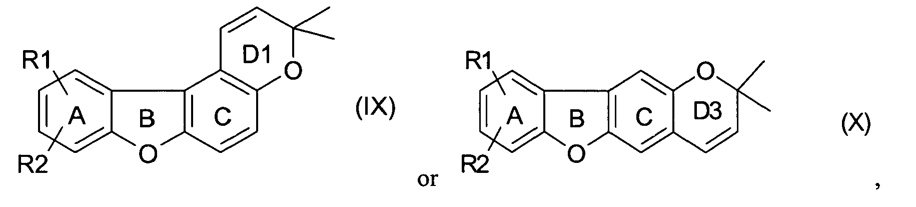 wherein R1 and R2 are as defined above, comprising:
(g2) suspending sodium hydride in a dry solvent, advantageously chosen from toluene, benzene or xylene, more advantageously toluene, under an inert atmosphere, and adding a compound of formula 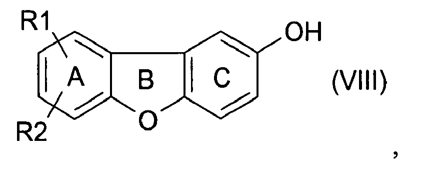
(h2) stirring at room temperature, advantageously between 10 and 45 minutes, more advantageously for 30 minutes;
(i2) adding chlorobutyne and potassium iodide and refluxing the suspension, advantageously between 24 hours and 7 days, more advantageously for three days,
(j2) removing the solvent, and purifying the residue by chromatography over silica gel in order to obtain a mixture of the compound of formula (IX) and of the compound of formula (X),
(k2) recrystallizing the mixture obtained in (j2) in methanol to obtain compound of formula (IX), or
(k3) purification of the compound of formula (X) by reverse phase HPLC.

In a preferred embodiment of the methods according to the invention, R1 and R2 represent an hydrogen atom.

Several types of derivatives of compounds of formula (IX) may be obtained by methods well known in the art. For instance, a catalytic osmium oxidation of the double bond of the pyran ring, using *N*-methylmorpholine *N*-oxide, permits to generate cis diols derivatives, such as compound **12** described above. Besides, a permanganate oxidation of compound of formula (IX) leads to the isolation of an α-hydroxy ketone derivative, such as compound **13** described above. Reduction of such an α-hydroxy ketone derivative using sodium borohydride allows for the preparation of a *trans* diol derivative, such as compound **15** described above. Alcohol functions may be alkylated to generate carbonate derivatives, such as compounds **18** to **20** described above. Cyclic carbonates derivatives, such as compound **22** described above, may be obtained upon treatment of cis-diol derivatives with *N,N'*-carbonyl diimidazole in 2-butanone. A catalytic palladium hydrogenation of compound of formula (IX) leads to the formation of an hydrogenated compound such as compound **16.** Any other modification of compound of formula (IX) by methods known by one skilled in the art may be performed.

The invention further concerns a pharmaceutical composition comprising at least one compound as described above, with an acceptable carrier.

The composition may be in the form of a solid, liquid or aerosol. Examples of solid compositions include tablets, gelatin capsules, powders, granules, suppositories, creams, and implantable dosage units. Examples of liquid compositions include aqueous solutions, isotonic saline solutions, injectable solutions, emulsions, syrup, and elixir. Examples of aerosol formulations include inhaler formulations for administration to the lungs.

When a solid composition is prepared in the form of tablets, the main active ingredient is mixed with a pharmaceutical vehicle such as gelatin, starch, lactose, magnesium stearate, talc, gum arabic and the like. The tablets may be coated with sucrose or with other suitable materials, or they may be treated in such a way that they have a prolonged or delayed activity and they continuously release a predetermined amount of active principle.

A preparation in gelatin capsules is obtained by mixing the active ingredient with a diluent and pouring the mixture obtained into soft or hard gelatin capsules.

A preparation in the form of a syrup or an elixir may contain the active ingredient together with a sweetener, an antiseptic, and also a taste enhancer and a suitable coloring agent.

The water-dispersible powders or granules may contain the active ingredient mixed with dispersing agents or wetting agents, or suspending agents, and with flavor correctors or sweeteners.

For rectal administration, suppositories are used which are prepared with binders which melt at rectal temperature, for example cocoa butter or polyethylene glycols.

For aerosol administration, particularly to the respiratory tract, the compound will generally have a small particle size for example of the order of five (5) microns or less. Such a particle size may be obtained by means known in the art, for example by micronization. The active ingredient is provided in a pressurized pack with a suitable propellant such as a chlorofluorocarbon (CFC), for example, dichlorodifluoromethane, trichlorofluoromethane, or dichlorotetrafluoroethane, or carbon dioxide or other suitable gas. The aerosol may conveniently also contain a surfactant such as lecithin. The dose of drug may be controlled by a metered valve.

The composition may be administered by any standard route of administration, including topical, oral, rectal, nasal or parenteral (for example, intravenous, subcutaneous, or intramuscular) routes. Depending on the administration route, the composition will be formulated in any suitable form known by one skilled in the art. For instance, tablets, gelatin capsules, powders, granules, syrups and elixirs may be administered orally, therapeutic creams may be administered topically; implantable dosage units may be administered locally, for example, subcutaneously, and aerosol are particularly suitable for administration in the respiratory tract. In addition, the composition may be incorporated into sustained release matrices such as biodegradable polymers, the polymers being implanted in the vicinity of where delivery is desired. Advantageously, the composition is administered by oral route.

The dosage of the composition will depend on the condition being treated, the particular composition used, and other clinical factors such as weight and condition of the patient, and the route of administration.

In certain embodiment, a pharmaceutical composition according to the invention may further comprise at least one other antibiotic agent. As used herein, an "antibiotic agent" may be any agent able to prevent the growth of or to destroy some bacteria. In particular, when bacteria are *Mycobacterium tuberculosis* bacteria, usual antibiotic agents used for the treatment of tuberculosis include isoniazid (INH), rifampin (RIF), pyrazinamide, ethambutol, streptomycin, rifabutine, the aminoglycosides (Kanamycine/Amykacine Capréomycine), Acide aminosalicylique, Ethionamide, D-Cycloserine or Fluoroquinolones, (Ofloxacine, moxifloxacine, levofloxacine). In a preferred embodiment, when a pharmaceutical composition according to the invention is directed to the treatment of tuberculosis, it may thus further comprise at least one of these antibiotics agents. Preferably, such a pharmaceutical composition comprises a combination of several of these antibiotic agents, such as that used in current short course chemotherapy (DOTS), which consists of a combination of isoniazid (INH), rifampin (RIF), pyrazinamide and ethambutol or streptomycin.

The invention also concerns a compound as described above, including any pharmaceutically acceptable salt, isomer, enantiomer, or diastereoisomer thereof, or mixture thereof, or a pharmaceutical composition as described above, as a medicament. Said medicament may be formulated in any form and administrated by any route described above.

The compounds according to the invention are particularly suitable for use as medicament because of their low cytotoxicity on mammalian cells. In particular, compounds according to the invention were shown by the inventors to display a low cytotoxicity on macrophages and pneumocytes, which are the target cell types of *Mycobacterium tuberculosis.*

In certain embodiments of the invention, said medicament is directed to the treatment of fungal or bacterial infections. In particular, said medicament may be directed to the treatment of infections by *Candida albicans,* which is one of the most frequent pathogenic fungus of humans, *Aspergillus, Cryptococcus,* or *Pneumocystis.*

Concerning bacterial infections, a medicament according to the invention is preferably directed to the treatment of a mycobacterial infection, including *Mycobacterium tuberculosis, Mycobacterium avium* and *Mycobacterium leprae* infections, or of infection by a bacterial strain that shares similarities in its cell wall composition with mycobacteria. According to the invention, a bacterial strain that shares similarities in its cell wall composition with mycobacteria is a bacterial strain that contains mycolic acid, such as actinomycetes, in particular *Nocardia asteroides.* Advantageously, a bacterial strain that shares similarities in its cell wall composition with mycobacteria is *Nocardia asteroides.* In addition, when a medicament according to the invention is preferably directed to the treatment of infection by a bacterial strain that shares similarities in its cell wall composition with mycobacteria, in particular *Nocardia asteroides,* said medicament preferably comprises compound 9 or compound 16 according to the invention.

Preferably, a medicament according to the invention is directed to the treatment of an infection caused by a *Mycobacterium tuberculosis* strain. *Mycobacterium tuberculosis* strains against which compounds according to the invention display an inhibitory activity notably include *Mycobacterium tuberculosis* H37Rv, H37Ra, the particularly virulent strain Beijing (GC1237), and ionazide resistant strain 2003/1762 and rifampin resistant strain 2004/0499.

In the case of tuberculosis, inappropriate regimens or inadequate compliance to current short course chemotherapy (DOTS) has entailed the rise of drug resistant strains of *Mycobacterium tuberculosis,* and even to multiple drug resistant strains (MDR-TB). For instance, *Mycobacterium tuberculosis* strains that are resistant to isoniazid or rifampin or both have been described. Compounds **9** and **16,** as described above, were shown to have the same effect on non resistant *Mycobacterium tuberculosis* strains and on isoniazid or rifampin resistant *Mycobacterium tuberculosis* strains (see Table 3). In certain embodiments, a medicament according to the invention is thus directed to the treatment of infections by a *Mycobacterium tuberculosis* strain that is known to be resistant to at least one antibiotic agent, such as ionazide resistant strain 2003/1762 and rifampin resistant strain 2004/0499.

In a preferred embodiment, a medicament according to the invention is used in combination with at least one other antibiotic agent. In particular, when bacteria are *Mycobacterium tuberculosis* bacteria, usual antibiotic agents used for the treatment of tuberculosis include isoniazid (INH), rifampin (RIF), pyrazinamide, ethambutol, streptomycin, rifabutine, the aminoglycosides (Kanamycine/Amykacine Capréomycine), Acide aminosalicylique, Ethionamide, D-Cycloserine and Fluoroquinolones, (Ofloxacine, moxifloxacine, levofloxacine). In a preferred embodiment, when a pharmaceutical composition according to the invention is directed to the treatment of tuberculosis, it may thus further comprise at least one of these antibiotics agents. Preferably, such a pharmaceutical composition comprises a combination of several of these antibiotic agents, such as that used in current short course chemotherapy (DOTS), which consists of a combination of isoniazid (INH), rifampin (RIP), pyrazinamide and ethambutol or streptomycin.

Tuberculosis may remain in an individual in a latent asymptomatic form. However, such a latent tuberculosis infection may be reactivated in immunodeficient patients. In a particular embodiment, a medicament according to the invention is thus directed to the treatment of immunodeficient patients infected by mycobacterial pathogenic strain. As used herein, immunodeficient patients represent any patient whose immune system is not able to mount efficient immune responses to infections. Examples of immunodeficient patients include AIDS patients, or patients who are treated with immunosuppressive drugs, such as patients treated against autoimmune diseases or transplanted patients.

Having generally described this invention, a further understanding of characteristics and advantages of the invention can be obtained by reference to certain specific examples and figures which are provided herein for purposes of illustration only and are not intended to be limiting unless otherwise specified.

### DESCRIPTION OF THE FIGURE

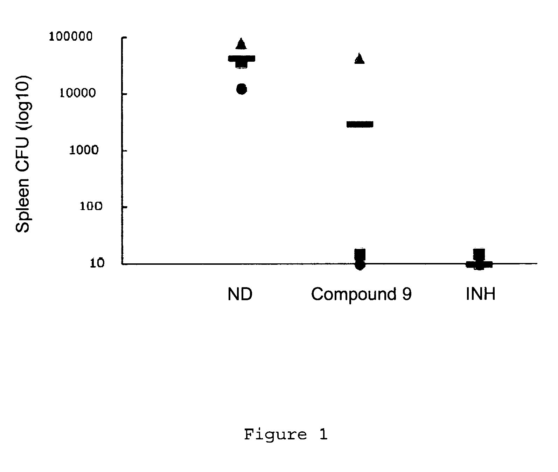

Figure 1. Bacterial load (CFU) in spleens of mice infected by M. tuberculosis H37Rv and treated with excipient alone (ND), compound 9 at 50 mg/kg/day (compound 9), or ionazide at 25 mg/kg/day (INH).

### Example 1

### Synthesis and analysis of compounds

### 1.1 Analysis

Melting points were determined on a hot stage Reichert microscope and were uncorrected. Mass spectra were recorded with a Nermag R-10-10C spectrometer using desorption-chemical ionisation (DCI-MS; reagent gas: NH3.). UV spectra (λmax in nm) were recorded in spectroscopic grade MeOH on a Beckman Model 34 spectrophotometer. IR Spectra (νmax in cm⁻¹) were obtained from potassium bromide pellets or sodium chloride films on a Perkin-Elmer 257 instrument.¹H NMR (™ [ppm], J [Hz]) spectra were run at 400 MHz and ¹³C spectra at 75 MHz, using Bruker AVANCE-400 and AC-300 spectrometers, respectively. All the structures were insured and the signals unambiguously assigned by 2D NMR techniques: ¹H-¹H COSY, ¹H-¹H NOESY, ¹³C-¹H HMQC and ¹³C-¹H HMBC. These experiments were performed using standard Bruker microprograms. Column chromatographies were performed on Merck silica gel 35-70 µM or 20-45 µM. 2-hydroxydibenzofuran (4) were purchased from Aldrich Chemical Company (Steinheim, Germany).

### 1.2 Synthesis and characteristics of obtained compounds

### 1.2.1 Preparation of 2-(1,1-dimethyl-propargyloxy)-dibenzofuran (5).

To a solution of 2-hydroxydibenzofuran **(4)** (16.20 mmoles, 3 g) in anhydrous acetonitrile (6 mL, dried over 4A molecular sieve) under argon and cooled using an ice-salt bath were added DBU (16.20 mmol, 1.68 mL) and copper chloride dihydrate (0.20 mol, 3 mg) followed by the addition of chlorobutyne 3 (16.20 mmol, 2.50 mL). After stirring for 5 hours at 0°C, the mixture was concentrated at reduced pressure and the residue was partitioned between water (20 mL) and toluene (20 mL). The organic fraction was washed successively with hydrochloric acid 1N (20 mL), sodium hydroxide 1N (20 mL), 1N sodium hydrogenocarbonate (20 mL) and brine (20 mL) before being dried over anhydrous magnesium sulfate. After filtration, the solvent was then evaporated to dryness under reduced pressure to give compound **5** as a pale yellow oil (3.40 g, 85 %) which was used without further purification.

IR (KBr) νmax cm⁻¹ : 3299, 2992, 2918, 2358, 2334, 1593, 1481, 1444, 1175, 1137, 747. UV. λ nm (log Σ) in methanol: 211 (4.06), 251 (3.59), 287 (3.49) ¹H (CDCl₃): 7.98 (dd, 1H, J = 9, 2, H-9); 7.81 (d, 1H, J = 2, H-1), 7,60 (dd, 1H, J = 9, 2, H-6); 7.50 (d, 1H, J = 9, H-3); 7.48 (td, 1H, J = 9, 2, H-7); 7.35 (td, 1H, J = 9, 2, H-8); 7.33 (d, 1H, J = 9, H-4); 2.60 (s, CH.C), 1.53 (s, 3H, 2 x CH₃). ¹³C (400 MHz, CDCl₃) :157.2 (C2); 153.1 (C5a); 151.4 (C4a); 127.5 (C7); 124.8 (C9a); 124.7 (2C, C9 + C8); 121.1 (C6); 114.6 (C4); 112.1 (C1); 111.9 (C9b); 111.7 (C3); 86.6 (HC.C), 74.3 (HC.C), 73.82 (C(CH₃)₃), 30.0 (2C, CH₃); *m*/*z* : = 251.[MH]⁺.

### 1.2.2 General synthesis procedure.

### Method A. Preparation of compounds 8-10 (see Scheme 1 below):

Under an inert atmosphere, sodium hydride (50% in oil 67.70 mmol, 3.25 g) was dissolved in dry toluene (250 mL) and 2-hydroxydibenzofuran (**4**) (27.14 mmol, 5.00 g) was slowly added. After 30 min of stirring at room temperature, chlorobutyne (3)36,47 (30.00 mmol, 3.10 g) and potassium iodide (30.00 mmol, 4.98 g) were added and the suspension was refluxed for three days. After removal of the solvent under reduced pressure, the residue was purified by chromatography over silica gel eluting with a mixture of cyclohexane/dichloromethane in proportions varying from 99.5-0.5 to 98-2 to yield the 1/10 mixture of compounds **8** and **9** (3.94 g, 58 %) as well as compound **10** (0.72 g, 11%). A recrystallisation of the above-mentioned mixture in methanol lead to compound **9** (1.40 g, 21%) as white needles.

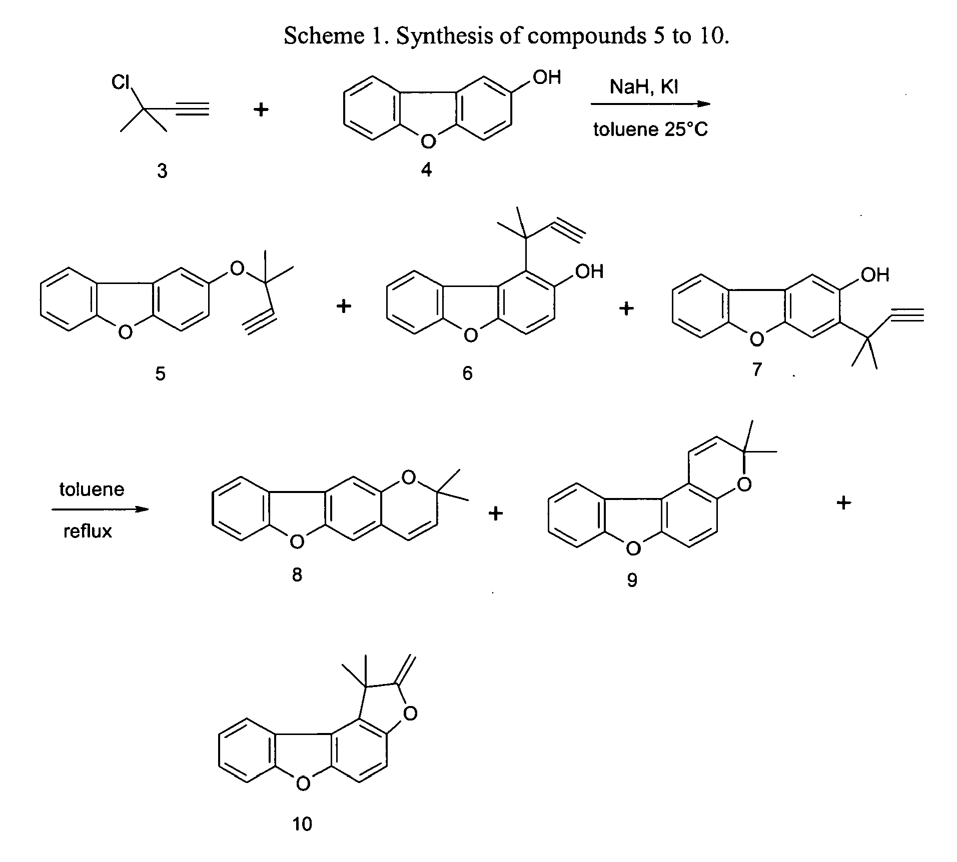

### Method B.

Compound **9** was also obtained, from ether propargylic **5** (20.00 mmol, 5.00 g) by heating in 1,3-dichlorobenzene (150 mL) during 12 hours, as white needles (1.60 g, 53 %) after purification by chromatography over silica gel (solvant: cyclohexane/dichloromethane 98/2 v/v).

**3,3-dimethyl-3*H*-benzofuro[3,2-*f*][1]benzopyran (9) :** mp : 101°C (from MeOH). IR (KBr) vmax cm⁻¹ : 2975, 1424, 1146, 810. UV. λ nm (log Σ) in methanol: 217 (6.82), 276 (6.37), 295 (6.67), 305 (6.73) ¹H (CDCl₃): 8.01 (dd, 1H, J = 8, 2, H-11); 7.57 (dd, 1H, J = 8, 1, H-8), 7,45 (td, 1H, J = 8, 2, H-9); 7.35 (m, 2H, H-6 and H-10); 7.11 (d, 1H, J = 9, H-1); 6.95 (d, 1H, J = 9, H-5); 5.85 (d, 1H, J = 9, H-2); 1.53 (s, 3H, CH₃); 1.38 (s, 3H, CH₃). ¹³C (400 MHz, CDCl₃) :157.1 (C7a); 151.2 (C6a); 148.6 (C4a); 132.2 (C2); 126.9 (C9); 124.5 (C11a); 123.0 (C11); 122.6 (C10); 120.65 (C11b); 119.4 (C1); 116.1 (C5); 115.8 (C11c); 111.9 (C8); 111.2 (C6); 75.8 (C3); 27.7 (CH₃); 27.5 (CH₃). *m*/*z* : = 251.[MH]⁺ .Anal. (C₁₇H₁₄0₂) : C, H, O.

### 1,2-dihydro-1,1-dimethyl-2-methylenebenzofuro[5,4-b]benzofuran (10) :

### White needles.

mp : 121°C (from MeOH). IR (KBr) νmax cm⁻¹: 2958, 2916, 2856, 2363, 2335. U.V. λ nm (log Σ) in methanol : 237 (3.37), 260 (3.02), 292, (3.22). ¹H (CDCl₃): 7,85 (dd, 1H, J=8, 1, H-10), 7.44 (dd, 1H, J = 8, 1, H-7), 7.34 (td, 1H, J = 8, 1, H-8), 7.24 (d, 1H, J = 9, H-5), 7.22 (dd, 1H, J = 8, 2, H-9), 6.91 (d, 1H, J = 9, H-4), 4.56 (d, 1H, J = 3, H-2a), 4.16 (d, 1H, J = 3, H-2b), 1.62 (s, 3H, CH₃), 1.53 (s, 3H, CH₃). ¹³C (CDCl₃) : 172.6 (C2), 157.2 (C6a), 152.6 (C5a), 151.7 (C3a), 127.3 (C8), 127.1 (C10a), 123.5 (C10), 122.5 (C9), 122.4 (C10b), 119.9 (C10c), 112.0 (C4), 111.9 (C7), 108.7 (C5), 82.0 (C2'), 45.0 (C1), 29.7 (CH₃), 28.6 (CH₃). *mlz* : = 251.[MH]⁺ Anal. (C₁₇H₁₄0₂) : C, H, O.

### 1.2.3 Oxidation of the mixture of dibenzofurans 8 and 9 (see Scheme2 below).

The mixture of the linear and angular pyranodibenzofuran **8** and **9** (1.98 mmol, 0.05 g) was added to a solution of osmium tetroxide (2.5 % in 2-methyl-2-propanol) (1.25 mL) and *N*-methylmorpholine *N*-oxide (1.98 mmol, 232.00 mg) in *ter*-butanol-tetrahydrofuran-water (10/3/1, v/v/v, 8.90 mL) and stirred at room temperature during 2 days. After addition of saturated aqueous sodium hydrogensulfite (20 mL) and stirring for 1h, the solution was then extracted with dichloromethane (5 x 20 mL). The organic layers were dried over anhydrous magnesium sulfate before being evaporated to dryness under reduced pressure. A flash chromatography (solvent cyclohexane/ ethyle acetate from 99/1 to 95/5 v/v) afforded **11** (0.10 g, 17 %) as white crystals and **12** (0.40 g, 72 %) as an amorphous white solid.

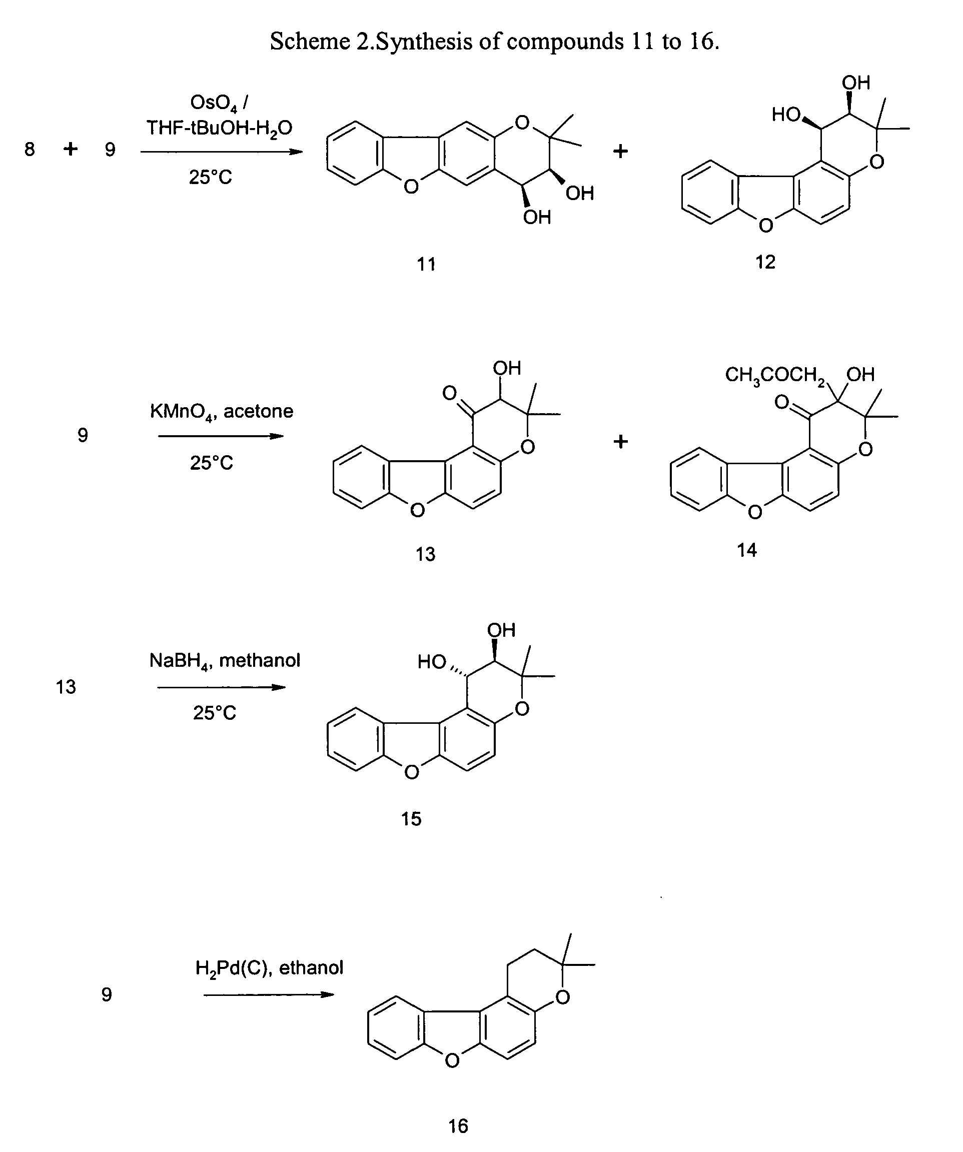

**(±)*cis*-3,4-dihydro-3,4-dihydroxy-2,2-dimethyl-2*H*-benzofuro[2,3-*g*]-[1]-benzopyran (11) :** mp: 206°C (from MeOH). IR (KBr) νmax cm⁻¹ : 3355, 2963, 2928, 1576, 1462, 1433, 1172, 750. U.V. λ nm (log Σ) in methanol :244 (5.57), 253 (5.67), 260 (4.26), 291 (5.69) , 316 (5.25), 326 (5.27), 347 (3.91). ¹H (CDCl₃) : 7.85 (dd, 1H, J = 8, 1, H-10), 7.75 (d, 1H, J = 1, H-5), 7.56 (dd, 1H, J = 8, 1 H-7), 7.45 (td, 1H, J = 8, 1, H-8 ), 7.37 (s, 1H, H-11), 7.31 (td, 1H, J = 8, 1, H-9), 4.95 (dd, 1H, J=9, 4, H-4), 3.78 (dd, 1H, J=9, 4, H-3), 2.84 (s, 1H, J=9, OH-4), 2.09 (s, 1H, J=9, OH-3), 1.45 (s, 3H, CH₃), 1.2 (s, 3H, CH₃). ¹³C (CDCl₃) :157.1 (C6a), 151.5 (C5a), 148.0 (C11a), 127.6 (C8), 125.4 (C4a), 124.1 (C10a) , 122.6 (C10b), 122.5 (C9), 120.9 (C10), 111.8 (C7), 111.1 (C5), 108.1 (C11), 77.9 (C2), 71.6 (C3), 66.5 (C4), 25.0 (CH₃), 23.3 (CH₃). *m*/*z* : = 285.[MH]⁺. Anal. (C₁₇H₁₆0₄): C, H, O.

**(±)*cis*-1,2-dihydro-1,2-dihydroxy-3,3-dimethyl-3*H*-benzofuro[3,2-*f*][1]benzopyran (12) :** IR (KBr) νmax cm⁻¹: 3493, 3331, 2929, 1429, 1038, 930. U.V. λ nm (log Σ) in methanol : 254 (5.84), 290 (5.90), 314 (5.43) , 325 (5.45). ¹H (CDCl₃) : 8.40 (dd, 1H, J = 8, 1, H-11), 7.56 (dd, 1H, J = 8, 1, H-8), 7.46 (td, 1H, J = 8, 1, H-9), 7.44 (d, 1H, J = 9, H-6 ), 7.36 (td, 1H, J = 8, 1, H-10), 6.97 (d, 1H, J = 9, H-5), 5.28 (dd, 1H, J = 9, 5, H-1), 3.92 (dd, 1H, J = 9, 5 H-2), 2.96 (d, 1H, J = 9, OH-1), 2.44 (d, 1H, J = 9, OH-2), 1.53 (s, 3H, CH₃), 1.39 (s, 3H, CH₃). ¹³C (CDCl₃) : 156.9 (C7a), 151.1 (C6a), 147.9 (C4a), 127.0 (C9), 124,5 (C11a), 122.4 (C11), 122.2 (C10), 119.8 (C11b), 117.1 (C5), 116.1 (C11c), 112.7 (C8), 111.4 (C6), 77.6 (C3), 71.8 (C2), 64.4 (C1), 23.4 (CH₃), 23.1 (CH₃). *m*/*z* : = 285.[MH]⁺ . Anal. (C₁₇H₁₆0₄): C,H,O

### 1.2.4 Preparation of compounds 13-14 (see Scheme 2).

A suspension of powered potassium permanganate (3.66 mmol, 0.58 g) in water (2.14 mL) was added drop-wise for 2 h to a solution of 9 (0.66 mmol, 0.17 g) in acetone (4.00 mL) at room temperature. The solvents were removed under reduced pressure and the residue was submitted to flash chromatography with cyclohexane/dichloromethane (from 90/10 v/v to 35/65 v/v) to afford compounds 13 (0.10 g, 55 %) and 14 (0.01 g, 5%).

### (±)2,3-dihydro-2-hydroxy-3,3-dimethyl-1H-benzofuro[3,2-f][1]benzopyran-1-one (13).

Amorphous white solid. IR (KBr) νmax cm⁻¹: 2485, 3101, 2976, 1680, 1440, 1083, 906, 743. U.V. λ nm (log Σ) in methanol : 251 (5.04), 309 (3.70), 311 (3.71), 338 (3.45) , 365 (3.55). ¹H (CDCl₃) : 9.05 (dd,1H, J = 7, 2, H-11), 7.73 (d,1H, J = 9, H-6), 7.57 (dd, 1H, J = 8, 1, H-8), 7.53 (td, 1H, J = 8, 2, H-9), 7.41 (ddd, 1H, J = 8, 7, 1, H-10), 7.05 (d,1H, J = 9, H-5), 4.57 (s, 1H, H-2), 4.05 (s, 1H, OH), 1.71 (s, 3H, CH₃), 1.29 (s, 3H, CH₃). ¹³C (CDCl₃) : 194.3 (CO), 157.5 (C7a), 157.1 (C6a), 150.6 (C4a), 128.4 (C9), 126.4 (C11), 123.2 (C11a), 122.6 (C10), 122.0 (C11b), 119.8 (C5), 117.4 (C8), 112.9 (C11c), 111.1 (C6), 88.2 (C3), 76.4 (C2), 26.8 (2XCH₃). *m*/*z* := 283.[MH]⁺. Anal. (C₁₇H₁₄0₄): C, H, O.

### (±)2,3-dihydro-2-hydroxy-3,3-dimethyl-2-(2-oxopropyl)-1Hbenzofuro[3,2f][1]benzopyran-1-one (14).

Amorphous white solid. IR (KBr) vmax cm⁻¹ : 3082, 2984, 2940, 1709, 1596, 1440, 1110. λ nm (log Σ) in methanol : 242 (6.12), 313 (6.27), 338 (6.01), 360 (7.16). ¹H (CDCl₃) : 8.86 (dd, 1H, J = 8, 1, H-11), 7.71 (d, 1H, J = 9, H-6), 7.58 (dd, 1H, J = 8, 1, H-8), 7.53 (td, 1H, J = 8,1, H-9), 7.40 (td, 1H, J = 8, 1, H-10), 7.02 (d,1H, J = 9, H-5), 4.79 (s, 1H, OH), 3.06 (s, 2H, CH2-CO-CH₃), 2.1 (s, 3H, CH2-CO-CH₃), 1.57 (s, 3H, CH₃), 1.41 (s, 3H, CH₃). ¹³C (CDCl₃) : 188.0 (CO), 169.4 (CH₂COCH₃), 157.7 (C7a), 155.4 (C6a), 151.0 (C4a), 128.8 (C9), 126.2 (C11), 123.3 (C11a), 123.0 (C10), 122.5 (C11b), 119.3 (C5), 117.0 (C8), 116,1 (C11c), 113.3 (C6), 84.3 (C3), 68.1 (C2), 45.6 (CH2COCH₃), 32.0 (CH2COCH₃), 23,7 (2XCH₃). *m*/*z* : = 339.[MH]⁺. Anal. (C₂₀H₁₈0₅) : C, H, O

### 1.2.5 Preparation of (±)trans-1,2,-dihydro-1,2-dihydroxy-3,3-dimethyl-3H-benzofuro[3,2-f][1]benzopyran (15) (see Scheme 2).

To a cold solution of 13 (1.10 mmol, 0.31 g) in methanol (12.00 mL) was added NaBH4 (8.80 mmol, 0.33 g). The mixture was stirred for 15 min at room temperature. The reaction was quenched by addition of water, and extracted by dichloromethane (2 x 5 mL). The organic layers were dried over magnesium sulfate and evaporated to dryness. The cis/trans mixture was purified by OPLC chromatography eluting with dichloromethane/ethylacetate (90/10 V/V) and allowed some pure compound 15 (0.08g, 26 %) to be isolated as amorphous white solid. IR (KBr) vmax cm⁻¹ : 3438, 2980, 2908, 2843, 2357, 1621, 1473, 1180, 813, 754 λ nm (log Σ) in methanol :221 (5.64), 202 (7.14), 207 (7.13), 254 (6.78), 260 (5.54), 290 (6.86), 325 (6.39). ¹H (CDCl₃) : 8.31 (dd, 1H, J = 8, 2, H-11), 7.55 (d, 1H, J = 8, H-8), 7.47 (td, 1H, J = 8,2, H-9), 7.45 (dd, 1H, J = 9, H-6), 7.35 (td, 1H, J = 8, 1 H-10 ), 6.97 (d, 1H, J = 9, H-5), 5.1 (dd, 1H, J = 7, 6, H-1), 3.88 (t, 1H, J = 6, H-2), 2.47 (d, 1H, J = 6, OH-2), 2.14 (d, 1H,J = 7, OH-1), 1,54 (s, 2X3H, 2XCH₃). ¹³C (CDCl₃) : 156.9 (C7a), 151.1 (C6a), 148.3 (C4a), 127.1 (C9), 124.2 (C11), 123.6 (C11a), 122.9 (C11b),122.5 (C10), 117.1 (C5), 116.1 (C11c), 112.8 (C8), 111.4 (C6), 77.8 (C3), 77.5 (C2), 69.8 (C1), 25.5 (2XCH₃). *m*/*z :* = 285.[MH]⁺. Anal. (C₁₇H₁₆0₄) : C, H, O.

**1,2-dihydro-3,3-dimethyl-3H-benzofuro[3,2-f][1]benzopyran (16).** To a solution of **9** (0.25 mmol, 0.06 g) in ethanol (15 mL) was added Pd/C (10 %, 0.007 g). The resulting mixture was flushed under H₂ (1 atm) at room temperature for 18 hours. After filtration, the solution was concentrated to dryness using a high vacuum pump and led to compound **16** (0.059 g, 96 %) as a white needles. mp : 124°C (from MeOH). IR (KBr) νmax cm⁻¹: 2971, 2920, 1638, 1420, 1266, 1168. UV. λnm (log ε) in methanol: 217 (3.68), 223 (3.77), 290 (3.87), 328 (3.85) .¹H (CDCl₃): 8.00 (dd, 1H, J = 8, H-11); 7.58 (d, 1H, J = 8, H-8), 7,48 (td, 1H, J = 8, 2, H-9); 7.37 (m, 2H, H-6 and H-10); 6.98 (d, 1H, J = 9, H-5); 3.28 (t, 2H, J = 4, H-1); 2.02 (t, 2H, J = 4, H-1); 1.42 (s, 6H, CH₃). ¹³C (400 MHz, CDCl₃) : 157.1 (C7a); 150.6 (C6a); 149.62 (C4a); 126.7 (C9); 125.59 300 (C11a); 122.7 (C11+C10); 122.5 (C11b); 123.0 (C11); 117.5 (C5); 115.7 (C11c); 111.9 (C8); 111.2 (C6); 74.1 (C3); 32.7 (C2); 26.9 (CH₃); 21.4 (C1). m/z : = 253.[MH]⁺. Anal. (C₁₇H₁₆O₂): C, H.

### 1.2.6 General procedure for the synthesis of esters 17-20 (see Scheme 3 below).

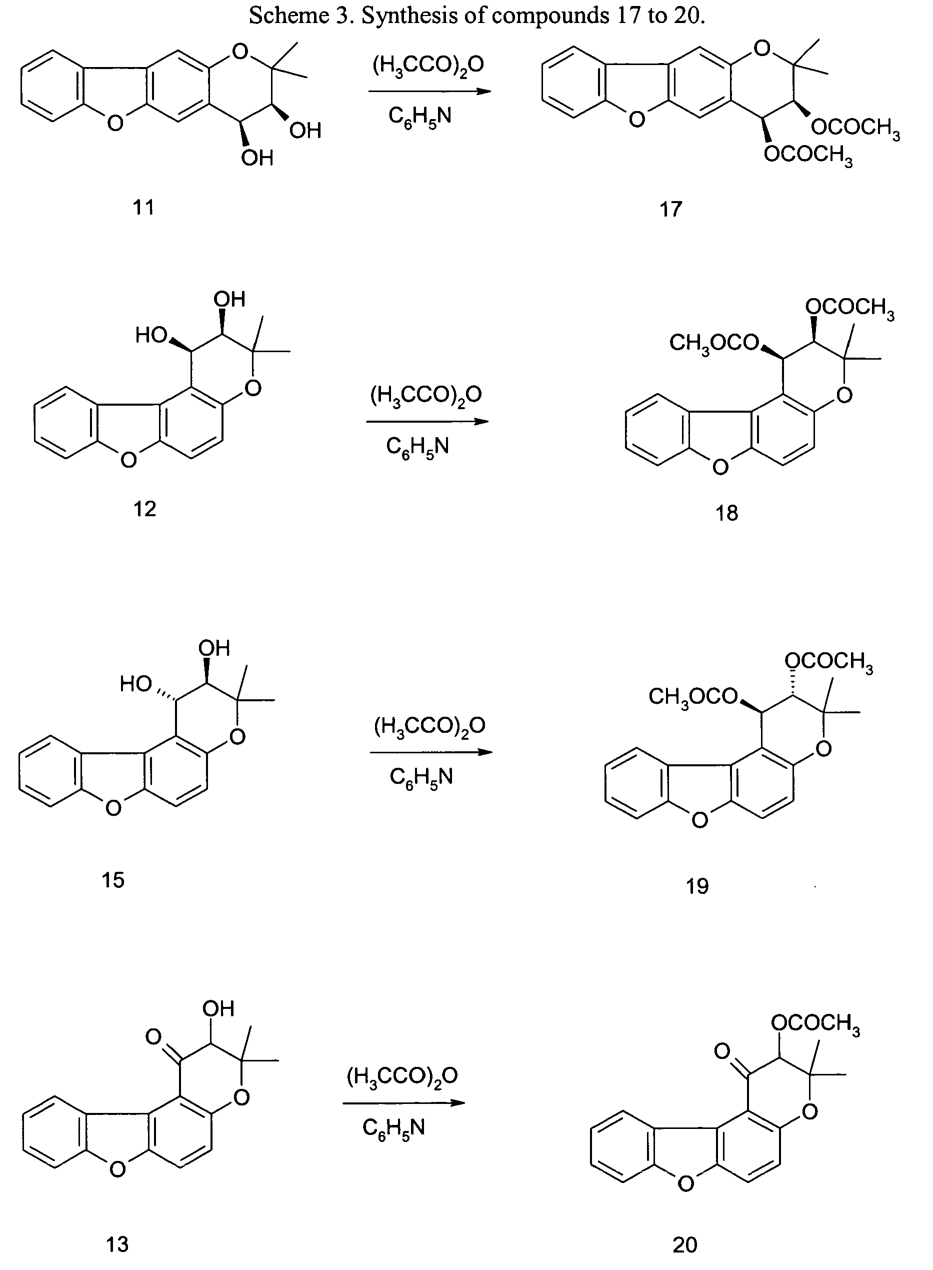

To a solution of 11-15 (0.17 mmol, 0.05 g) in dry pyridine (4.00 mL) was added an excess of acetic anhydride (4.00 mL). The reaction mixture was stirred during 2 days at room temperature. The resulting solution was poured on cold water. The precipitate was filtered, washed with water and then dried in a vacuum over phosphorus pentoxide to afford compounds 17-20 in the yield described below.

### (±)cis-3,4-diacetoxy-3,4-dihydro-2,2-dimethyl-2H-benzofuro[2,3-g]-[1]-benzopyran (17). 69 %.

Amorphous white solid. IR (KBr) νmax cm⁻¹ : 3438, 2980, 2908, 2843, 2357, 1621, 1473, 1180, 813, 754. λ nm (log Σ) in methanol : 209 (5.97), 245 (5.47), 254 (5.59), 260 (4.80), 291 (5.66), 315 (5.18), 326 (5.23). ¹H (CDCl₃) : 7.89 (d, 1H, J = 9, H-10), 7.53 (d, 1H, J = 9, H-7), 7.46 (t, 1H, J = 9, H-8), 7.43 (s, 1H, H-5), 7.38 (s, 1H, H-11), 7.32 (t, 1H, J = 9, H-9), 6.35 (d, 1H, J = 7, H-4), 5.40 (s, 1H, J = 7, H-3), 2.18 (s, 3H, OCOCH₃), 2.08 (s, 3H, OCOCH₃), 1.46 (s, 2X3H, 2XCH₃). ¹³C (CDCl₃) :170.6 (OCOCH₃), 157.2 (C6a), 150.9 (C5a), 148.8 (C11a), 127.7 (C8), 125.7 (C10a), 123.9 (C10b), 122.5 (C9), 120.9 (C10), 117.6 (C4a), 111.7 (C7), 109.9 (C5), 108.1 (C11), 77.1 (C2), 69.6 (C3), 66.1 (C4), 24.2 (OCOCH₃), 20.7 (2XCH₃). *m*/*z* = 369.[MH]⁺. Anal. (C₂₁H₂₀0₆): C, H, O

### (±)cis-1,2-diacetoxy-1,2-dihydro-3,3-dimethyl-3H-benzofuro[3,2-f][1]benzopyran (18). 70%.

White crystals mp. = 188.7°C (from CH₂Cl₂/MeOH). IR (KBr) νmax cm⁻¹ : 3722, 2986, 1734, 1374, 1240, 1188, 1093, 808. λ nm (log Σ) in methanol : 253 (5.28), 291 (5.35), 326 (5.03). ¹H (CDCl₃) :7.60 (dd, 1H, J = 8, 1,H-11), 7.53 (dd, 1H, J = 8, 1, H-8), 7.50 (d, 1H, J = 9, H-6), 7.44 (dd, 1H, J = 8, 1, H-9), 7.28 (td, 1H, J = 8,1, H-10), 7.01 (d, 1H, J = 9, H-5), 6.71 (d, 1H, J = 5, H-1), 5.45 (d, 1H, J = 5, H-2), 2.10 (s, 3H, OCOCH₃), 2.08 (s, 3H, OCOCH₃), 1.49 (s, 3H, CH₃), 1.45 (s, 3H, CH₃). ¹³C (CDCl₃) : 169.9 (O-CO-CH₃ C1), 156.8 (O-CO-CH₃ C2), 157.0 (C7a), 151.0 (C6a), 149.5 (C4a), 127.2 (C9), 123.5 (C11a), 123.0 (C11), 121.9 (C10), 123.0 (C11b), 117.5 (C5), 114.0 (C8), 112.0 (C6), 110.4 (C11c), 75.6 (C3), 71.7 (C1), 64.5 (C2), 21.5 (O-CO-CH₃ C1), 20.6 (O-CO-CH₃ C2), 25.9 (2XCH₃). *m*/*z* = 369.[MH]⁺. Anal. (C₂₁H₂₀0₆) : C, H, O

### (±)trans-1,2,-diacetoxy-1,2-dihydro-3,3-dimethyl-3H-benzofuro[3,2-f][1]benzopyran (19). 48%.

White crystals mp. = 259°C (from MeOH). IR (KBr) νmax cm⁻¹ : 3450, 3065, 2980, 1748, 1439, 1228. λ nm (log Σ) in methanol: 211 (5.23), 246 (5.05), 255 (5.08), 284 (5.16), 328 (4.53). ¹H (CDCl₃):7.60 (d,1H, J = 8, H-11), 7.56 (d,1H, J = 8, H-8), 7.53 (d, 1H, J = 9, H-6), 7.46 (t, 1H, J = 8, H-9), 7.31 (t, 1H, J = 8, H-10), 7.05 (d, 1H, J = 9, H-5), 6.28 (d, 1H, J = 3, H-1), 5.41 (d, 1H, J = 3, H-2), 2.13 (s, 3H, OCOCH₃), 2.08 (s, 3H, OCOCH₃), 1.49 (s, 3H, CH₃), 1.41 (s, 3H, CH₃). ¹³C (CDCl₃) : 170.7 (O-CO-CH₃ C1),169.7 (O-CO-CH₃ C2),156.9 (C7a), 151.0 (C6a), 149.4 (C4a), 127.2 (C9), 123.5 (C11a), 122.9 (C11), 121.4 (C11b), 121.6 (C10), 117.6 (C5), 113.7 (C8), 111.9 (C6), 110.8 (C11c), 75.4 (C3), 71.7 (C1), 67.7 (C2), 23.7 (O-CO-CH₃ C1), 23.6 (O-CO-CH₃ C2), 20.9 (2XCH₃). *m*/*z* = 369.[MH]⁺. Anal. (C₂₁H₂₀0₆) : C, H, O

### (±)2-acetoxy-2,3-dihydro-2-hydro-3,3-dimethyl-1H-benzofuro[3,2-f][1]benzopyran-1-one (20). 66%.

Amorphous white solid. IR (KBr) νmax cm⁻¹ : 3497, 2983, 2939, 1757, 1687, 1594. λ nm (log Σ) in methanol: 211 (2.40), 215 (2.46), 251 (2.67), 265 (2.46), 320 (2.76). ¹H (CDCl₃) : 9.1 (d, 1H, J = 8, H-11), 7.75 (d, 1H, J = 9, H-6), 7.58 (d, 1H, J = 8, H-8), 7.53 (td, 1H, J = 8, 1, H-9), 7.35 (dd, 1H, J = 8, 1, H-10), 7.07 (d,1H, J = 9, H-5), 5.7 (s, 1H, H-2), 1.71 (s, 3H, CH₃), 2.1 (s, OCOCH₃), 1.62 (s, 3H, CH₃), 1.58 (s, 3H, CH₃). ¹³C (CDCl₃) : 188.0 (CO), 169.4 (O-CO-CH₃),157.4 (C7a), 156.2 (C6a), 150.7 (C4a), 128.2 (C9), 126.8 (C11), 123.0 (C11a), 122.4 (C10), 122.2(C11b), 119.3 (C5), 117.0 (C8), 114.2 (C11c), 110.8 (C6), 80.7 (C2), 75.4 (C3), 26.0 (CH₃-COOH), 26.8 (2XCH₃). *m*/*z* = 325.[MH]⁺. Anal. (C₁₉H₁₆0₅) : C, H, O

### 1.2.7 Preparation of compounds 21-22 (see Scheme 4 below).

To a solution of 11 or 12 (0.10 mmol, 0.03 g) in 2-butanone (2.00 ml) was added N,N'-carbonyldiimidazole (0.50 mmol, 0.081 g). The solution was refluxed for 2 h under argon and after cooling, 5% aqueous sodium hydrogencarbonate (20 mL) was added. The solution was extracted with ethyl acetate (3 x 20 mL) and the combined organic layers were dried over anhydrous magnesium sulfate, filtered and evaporated under reduced pressure. The residue obtained was purified by chromatography over silica gel (solvent dichloromethane then dichloromethane/acetone 98/2 to 96/4 v/v).

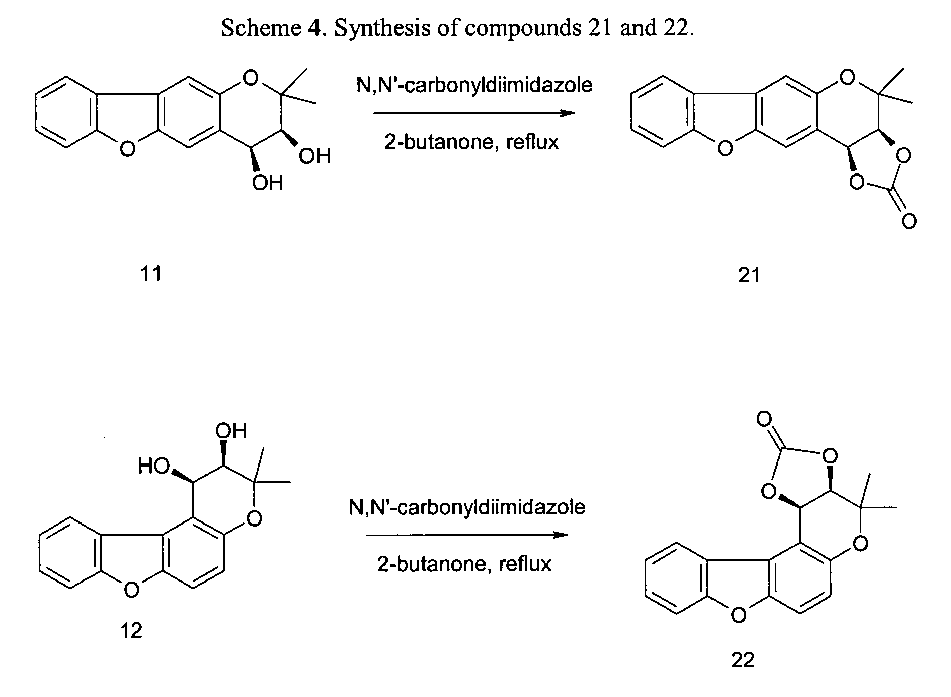

### (±)cis-4H-3a,4,12b-trihydro-2,2-dimethyl-1,3-dioxolo[4,5-h]benzofuro[3,2-g]benzopyran (21). 35 %.

Amorphous white solid .IR (KBr) νmax cm⁻¹ : 2918, 1794, 1175, 1072, 997. λ nm (log Σ) in methanol: 209 (5.65), 247 (5.17), 292 (5.20), 305 (4.93), 310 (4.92). ¹H (CDCl₃) : 8.1 (d, 1H, J = 8, 1, H-10), 7.8 (s,1H, H-5), 7.73 (s, 1H, H-11), 7.66 (d, 1H, J = 8, 1, H-7), 7.55 (dd, 1H, J = 8, 1, H-8), 7.38 (dd, 1H, J = 8, 1, H-9), 6.15 (d, 1H, J = 8, H-4), 5.13 (d, 1H, J = 8, H-3), 1.45 (s, 3H, CH₃), 1.15 (s, 3H, CH₃). ¹³C (CDCl₃) :159.5 (OCOO), 157.4 (C6a), 152.8 (C5a), 148.7 (C11a), 128.3 (C8), 127.4 (C10a), 123.5 (C10b), 122.9 (C9), 121.2 (C10), 116.2 (C4a), 112.3 (C7), 111.9 (C5), 109.8 (C11), 78.9 (C2), 75.3 (C3/C4), 30.9 (2XCH₃). *mlz* = 311.[MH]⁺. Anal. (C₁₈H₁₄0₅) : C, H, O.

### (±)cis-1,2-dihydro-3,3-dimethyl-1,3-dioxolo[4,5-h]benzofuro[3,2-f][1]benzopyran (22). 30%.

white needles. m.p. = 215°C (from MeOH). IR (KBr) νmax cm⁻¹ : 2990, 2933, 1802, 1626, 1175, 1029, 745. λ nm (log Σ) in methanol: 219 (5.95), 253 (5.23), 262 (4.60), 291 (5.32), 312 (4.99). ¹H (CDCl₃) : 8.06 (dd, 1H, J = 8,1, H-11), 7.59 (dd, 1H, J = 8, 1,H-8), 7.55 (d, 1H, J = 9, H-6), 7.51 (td, 1H, J = 8, 1, H-9), 7.39 (td, 1H, J = 8, 1, H-10), 7.02 (d, 1H, J = 9, H-5), 6.23 (d, 1H, J = 8, H-1), 4.88 (d, 1H, J = 8, H-2), 1.6 (s, 3H, CH₃), 1.4 (s, 3H, CH₃). ¹³C (CDCl₃) : 157.1 (O-CO-O), 153.8 (C7a), 151.3 (C6a), 148.5 (C4a), 127.8 (C9), 123.5 (C11a), 123.0 (C11), 122.9 (C11b), 122.7 (C10), 117.6 (C5), 114.7 (C8), 111.9 (C6), 109.7 (C11c), 78.8 (C3), 74.9 (C2), 70.4 (C1), 22.8/22.5 (2XCH₃). *m*/*z =* 311.[MH]⁺. Anal. (C₁₈H₁₄0₅) : C, H, O.

### Example 2

### Biological activity of synthesized compounds

The biological activities of pyranodibenzofuran derivatives **9-22** were investigated on a wide range of bacteria. The antimycobacterial activity was screened on the fast growing saprophyte *Mycobacterium smegmatis* mc²155 and on the virulent strain *Mycobacterium tuberculosis* H37Rv for all synthesized compounds. This was achieved using a liquid culture, which was then plated on solid media to determine the exact number of colony-forming units (CFU). The Minimal Inhibitory Concentration (MIC₉₉) is defined as the amount of compound required for > 99 % inhibition of bacterial growth (Table 1). Results of the most promising compounds on various strains of the *M. tuberculosis* complex, including the more virulent *Beijing* strain and drug resistant ones are shown in Table 2. To further assess the specificity of this new series, compounds were also screened for activity against a whole range of bacteria and fungi as well as for cytotoxicity (Tables 3 and 4).

### 2.1 Antifungal activity of synthesized compounds

The antifungal activity of compounds **5, 9,** and **11-22** against *Candida albicans* was analyzed by the measure of the MIC₉₉ concentration (see Table 1).

**Table 1. In Vitro Activity against Candida albicans of pyranodibenzofuran derivatives**

| Compound | MIC₉₉ (*ug*/mL) *C. albicans* |
|---|---|
| 5 | ND |
| 9 | > 600 |
| 10 | > 300 |
| 11 | 27 |
| 12 | > 160 |
| 13 | > 330 |
| 14 | 150 |
| 15 | > 300 |
| 16 | ND |
| 17 | > 400 |
| 18 | > 300 |
| 19 | > 300 |
| 20 | > 280 |
| 21 | > 400 |
| 22 | ND |
| INH | ND |
| Miconazole | 0.25-60 |

| | |
|---|---|
| ND: Not determined Data are representative of three independent experiments and standard deviation is within 10 % of the MIC₉₉. | |

Some of the synthesized compounds thus display some antifungal activity, particularly compounds **11** and **14,** although this activity is lower than that of the reference molecule miconazole.

### 2.2 Antibacterial activity of synthesized compounds

The antibacterial activity of compounds **5, 9,** and **10-22** was also investigated on different types of bacteria.

### 2.2.1 Bacterial strains.

*Escherichia coli (CIP 100631), Corynebacterium jeikeium* (ATCC43734), *Nocardia asteroides* (ATCC19247), were obtained from the Collection de l'Institut Pasteur, Paris, France. *Streptomyces coelicolor (M145), albus, avidini, avermitilis (ATCC31267); Pseudomonas aeruginosa* (13C3104), *Enterococcus faecalis* (14C1104), *Pneumococcus pneumoniae* (15C5004), *Staphylococcus aureus* (18C2204) strains were isolated from patients and kindly provided by Philippe Mazodier, Laurent Marsollier and GC1237 Beijing strains from Dr. Carlos Martin. Resistant strains (2003/1762 and 2004/0499) were obtained from Dr. Véronique Vincent (Laboratoires de référence des Mycobactéries, Institut Pasteur, Paris, France).

### 2.2.2 Antibacterial activity of compounds 5, 9, 11, 12, 15, 16 and 19 on Mycobacterium smegmatis MC²155 and on Mycobatecterium tuberculosis H37RV

Effect of the compounds was assessed on mycobacterial strains *in vitro* growth in liquid medium. Compounds were solubilized in DMSO and 100 µl of the appropriate dilution were added to a 5 ml culture with predetermined mycobacterium inocula. For the assays on the rapid growing mycobacterium *Mycobacterium smegmatis,* optical density of the culture in Luria-Bertani 410 medium at 595nm was measured after two-day's growth at 37°C. The effect on *Mycobacterium tuberculosis* strains and MDR-TB was monitored after three weeks growth in Middlebrook 7H9 broth at 37°C. Quantification of the mycobacteria was carried out by plating on 7H11 agar serial 5-fold dilutions in medium, and colony forming units (CFUs) ascertained after 2 weeks growth. The minimal inhibitory concentration (MIC) given is the concentration of compound for which 99% of the bacterial growth is inhibited.

**Table 2. In vitro Activity against M. smegmatis mc²155 and M. tuberculosis H37Rv of pyranodibenzofuran derivatives**

| N° (compound) | ClogP | MIC₉₉ (µg/mL) on *M. smegmatis* | MIC₉₉ (µg/mL) on *M. tuberculosis*^{a} |
|---|---|---|---|
| **5** | 5.3 | 12 | NI |
| **9** | 5.5 | 5 | 5 |
| **11** | 3.1 | 60 | NI |
| **12** | 3.4 | 30 | 60 |
| **15** | 3.4 | 30 | NI |
| **16** | 5.6 | 1 | 5 |
| **19** | 4.8 | 180 | NI |
| **INH** | -0.7 | 13 | 0.1 |

| | | | |
|---|---|---|---|
| MIC is the minimal concentration (µg/mL) of a compound resulting in a 99% decrease in the number of colony forming units of mycobacterial cultures compared to control (DMSO only). NI : No inhibition observed at the highest concentration tested (1 mg/mL). Data are representative of three independent experiments and standard deviation is within 10% of the MIC₉₉. LogP (ClogP) represents hydrophobicity and was determined using the ChemDrawPro software. a MIC of compound 9 and 16 were also determined by Bactec 460 (Becton Dickinson) method as previously described and are 10µg and 5 µg respectively. | | | |

From the results shown in table 2, an inhibitory activity on *M. smegmatis* mc² 155 of the 2,2-dimethyl-propargylic ether **5** (MIC₉₉ of 12 µg/mL), equivalent in this test to the control antibiotic isoniazid (INH), was found. Of even more interest, the 3,3-dimethyl-3*H*-benzofuro[3,2-*f*][1]-benzopyran **9** and its reduced analogue **16** with an MIC₉₉ of 5 µg/mL, are actually more potent than INH. The three diol-bearing compounds **11, 12** and **15,** derived from a controlled oxidation of compounds **8** or **9**, exhibited a lower level of activity ranking between 30 to 60 µg/ml.

### 2.2.3 In vitro antibacterial activity of compounds 5, 9 and 16 on different Mycobacterium tuberculosis complex strains

Compounds **5, 9** and **16** were then tested on strains of the complex *M. tuberculosis,* including H37Rv and the more virulent *Beijing.*

Effect of compounds on mycobacterial strains of the *M. tuberculosis* complex, including H37Rv and the more virulent *Beijing,* was assessed on *in vitro* growth in liquid medium. Compounds were solubilized in DMSO and 100 µl of the appropriate dilution were added to a 5 ml culture with predetermined mycobacterium inocula. The effect on *M. tuberculosis* strains and resistant strains was monitored after a three week growth in Middlebrook 7H9 culture (Difco, Sparks MD, USA) supplemented with oleic acid-albumindextrose-catalase (OADC, Difco, Sparks MD, USA) at 37°C. Quantification of the mycobacteria was carried out by plating on Middlebrook 7H11 medium (Difco, Sparks MD, USA) supplemented with OADC and colony forming units (CFUs) ascertained after a 2 week growth. The minimal inhibitory concentration (MIC₉₉) given is the concentration of compound for which 99% of the bacterial growth is inhibited.

Results are shown in the following Table 3.

**Table 3. In vitro activity of compounds 5, 9 and 16 against a whole range of mycobacteria**

| Mycobacterial strain of M. tuberculosis complex | | MIC₉₉ (µg/mL) | | | |
|---|---|---|---|---|---|
| | | 5 | 9 | 16 | INH |
| M. tuberculosis | H37Rv | > 500 | 5 | 5 | 0.1 |
| | Beijing (GC1237) | > 500 | 2 | 10 | 0.1 |
| | H37Ra | > 500 | 10 | 10 | 1 |
| | INH resistant (2003/1762) | ND | 5 | 1 | 5 |
| | RIF resistant (2004/0499) | ND | 5 | 1 | 0.1 |
| | M. bovis BCG (Pasteur) | > 500 | 10 | 10 | 1 |
| | M. microti (MYC-942272) | > 500 | 1 | ND | 0.1 |

| | | | | | |
|---|---|---|---|---|---|
| ND : Not Determined INH: ionazide, RIF: rifampin | | | | | |

Compound **9** as well as the reduced benzopyran **16** have a good inhibition activity on the growth of H37Rv and the more virulent *Beijing mycobacterium tuberculosis* strains. The corresponding MIC₉₉, which are less than 5 µg/mL, are indeed comparable to that of first-line anti-TB drugs such as ionazide.

In addition, exactly the same MIC₉₉ was found on RIP and INH resistant strains as on sensitive H37Rv strain (Table 3), indicating that compounds **9** or **16** could be particularly valuable for the treatment of drug-resistant *M. tuberculosis* strains.

### 2.2.4 In vitro antibacterial activity of compound 9 on bone marrow macrophages infected by Mycobacterium tuberculosis H37Rv

The activity of compound **9** was also evaluated in vitro on murine bone marrow macrophages infected by M. tuberculosis H37Rv.

Macrophages were cultured in 96 wells microplates at a 2.10⁵ macrophages/mL concentration for 7 days, and were then infected by *M. tuberculosis* H37Rv with a 1/10 ratio. After 2 hours, infected macrophages were washed and treated with different concentrations of compound **9** (respectively 50, 10, 5, and 1 µg/mL) for 24 or 48 hours. Macrophages were then lysed using triton X-100 at 0.1% in PBS. To count bacteria able to grow, the lysates were diluted and seeded on 7H11 culture medium. The enumeration of colony forming units (CFU) was determined after 4 to 6 weeks.

Results are shown in the following Table 4.

**Table 4. In vitro antibacterial activity of compound 9 on bone marrow macrophages infected by Mycobacterium tuberculosis H37Rv**

| Compound 9 concentration (µg/mL) | CFU | |
|---|---|---|
| | 24 hours treatment | 48 hours treatment |
| 50 | 1750 ± 1500 | 3800 ± 497 |
| 10 | 1500 ± 577 | 10000 ± 7360 |
| 5 | 3000 ± 816 | 2875 ± 250 |
| 0 | 4000 ± 1414 | 17500 ± 12583 |

Despite the presence of somewhat large standard deviations (50 µg/mL at 24 hours, 0 and 10 µg/mL at 48 hours), which are probably due to the low solubility of compound **9,** results of table 4 clearly indicate that compound **9** is able to significantly reduce the bacterial load in vitro in infected bone marrow macrophages (60% reduction of bacterial load for 10µg/mL at 24 hours).

### 2.2.5 In vivo antibacterial activity of compound 9 on mice infected by Mycobacterium tuberculosis

Compound **9** in vivo antibacterial activity on mice infected by *Mycobacterium tuberculosis* H37Rv was also assessed.

C57BL/6 and BalbC mice were infected by aerosol route with a bacterial load of 100 CFU each. Every day during 3 weeks, mice were fed with compound 9 (100, 50 or 25 mg/kg/day) in 200 µL of corn oil (Sigma) or in hydroxypropylcyclodextrine (HPCD). The treatment with compound **9** was started either on the day of the infection, or 3 weeks post infection. Mice were then sacrificed 30 days after and the bacterial load was assessed by seeding of organs lysates and CFU enumeration.

Results are shown in Figure 1 for mice treated from the day of infection with the excipient alone (ND), compound **9** at 50 mg/kg/day or ionazide at 25 mg/kg/day. (i.e. 0.5 mg/mouse/day).

At 30 days post infection, no bacteria was detected in 2 out of 3 mice treated with compound **9,** indicating that compound **9** is able to inhibit mycobacterium tuberculosis growth in vivo in mice.

### 2.2.6 Antibacterial activity of compounds 9 and 16 on Gram-positive, Gram-negative and other bacterial strains

To further assess its specificity, effect of compounds **9** and **16** was also assessed in *vitro* growth in liquid medium on a whole range of bacteria (*Corynebacterium jeikeium, Escherichia coli, Enterococcus faecalis, Klebsiella oxytoca, Nocardia asteroides, Staphylococcus aureus, Pseudomonas aeruginosa and Streptomyces).* Compounds were solubilized in dimethylsulfoxide (DMSO) and 100 µl of the appropriate dilution were added to a 5 ml culture with predetermined bacterium inoculum. Quantification of the bacteria was carried out by plating on appropriate agar serial 5-fold dilutions in medium, and colony forming units (CFUs) were ascertained after 24 to 48 hours according to the strains.

**Table 5. Antibacterial activity of compounds 9 and 16 on other bacteria**

| Strains | | MIC₉₉ (µg/mL) | |
|---|---|---|---|
| Compounds | | 9 | 16 |
| Gram-negative | *Escherichia coli* | > 600 | > 500 |
| | *Klebsiella oxytoca* | > 600 | ND |
| | *Pseudomonas aeruginosa* | > 500 | > 500 |
| Gram-positive | *Staphylococcus aureus* | > 100 | ND |
| | *Enterococcus faecalis* | > 500 | > 500 |
| | *Pneumococcus pneumoniae* | > 500 | ND |
| Actinomycetes | *Corynebacterium jeikeium* | > 150 | > 150 |
| | *Streptomyces Subspec* | > 500 | ND |
| | *Nocardia asteroides* | 12,8 | 15 |
| Fungus | *Candida albicans* | > 600 | ND |

The general antibacterial screening shown in Table 5 demonstrates that compound **9** and its closely related analogue **16,** like all the others described here, have no effect on Gram-positive and Gram-negative bacteria at the maximum concentration tested. Among the actinomycetes, a significant inhibitory effect was observed on *Nocardia asteroides.* Altogether these results suggest a very specific biological target within mycobacteria for these pyranodibenzofuran derivatives.

### 2.3 Cytotoxicity of synthesized compounds

The cytotoxicity of compounds **5, 9,** and **10-21** was determined in L1210 cells using the MTA (Microculture Tetrazolium Assay) test.

Results are indicated in the following Table 5.

**Table 5. cytotoxicity of compounds 5, 9, and 10-21 on L1210 cells**

| Compound | IC₅₀ (µg/mL) | SI |
|---|---|---|
| 5 | ND | ND |
| 9 | > 14 | > 2.8 |
| 10 | 3.705 | < 0.06175 |
| 11 | 12.996 | < 0.4332 |
| 12 | 1.3 | < 0.0013 |
| 13 | 5.0445 | < 0.0050445 |
| 14 | 18.981 | 0.6327 |
| 15 | 12.3405 | < 0.0123405 |
| 16 | ND | ND |
| 17 | 8.5215 | < 0.0085215 |
| 18 | 9.348 | < 0.009348 |
| 19 | 5.5005 | < 0.0055005 |
| 20 | 28.5 | < 0.0285 |
| 21 | 12.8535 | < 0.0128535 |
| 22 | ND | ND |
| INH | 14 | 2.8 |
| miconazole | ND | ND |

| | | |
|---|---|---|
| SI = IC₅₀/MIC₉₉ | | |

These results show that compounds **9, 11, 14, 15, 20** and **21** have a toxicity on eukaryotic cells, equivalent than INH.

The cytotoxicity of compounds **9** and **16** was further assessed in various mammalian cell lines using the MTT test. RAW and A549 cell lines were maintained in RPMI 1640 medium without L-GLU supplemented with 10% Fetal calf serum (FCS), penicillin-streptomycin solution 1 % at 37 °C in air with 5 % CO2. VERO cell lines were maintained in D-MEM medium without L-GLU supplemented with 10% Fetal calf Serum (FCS), penicillin-streptomycin solution 1 % at 37 °C in air with 5 % CO₂. Proliferating cells were seeded for 96-well microtitration plates at a density of 5. 10⁵ cells/mL which were further incubated for 24 h at 37°C under 5 % CO₂ in air before each assay. Various concentrations of solutions of **9** and **16** (10 µl/well) in 1.25 % dimethylsulfoxide (DMSO)) were added and then incubated for 24 hours under the above conditions. At the end of incubation, 20 µl of dimethylthiazolyl diphenyl tetrazolium bromide solution (MTT) (7.5 mg/ml) was then added into each well and further incubated for 4 h at 37°C to allow the formation of formazan. The crystals of formazan were then dissolved with 100 µl of an acidic solution (Sodium dodecyl sulfate (SDS) 10 %, HCl 10 mM). The optical density of each well was measured at 595 nm using a Multiwell plate reader. The values given are the average means of six replicates. The 50 % inhibition concentration was determined by curve fitting.

Results are indicated in the following Table 6.

**Table 6. cytotoxicity of compounds 9 and 16**

| Cell type | IC₅₀ (µg/mL) | SI | IC₅₀ (µg/mL) | SI |
|---|---|---|---|---|
| | **9** | | **16** | |
| VERO cells | 80 | 15 | 100 | 20 |
| Macrophages | 80 | 15 | > 100 | > 20 |

| | | | | |
|---|---|---|---|---|
| SI = IC₅₀/MIC₉₉ | | | | |

These results indicate that compounds **9** and **16** display a low cytotoxicity on mammalian VERO cells (IC₅₀ > 50 µg/ml), as well as on macrophages. In all cases, the observed IC₅₀ is higher than 50 µg/mL.

### 2.3 Conclusions

The results presented in the examples show that several of the synthesized compound display some antifungal *activity in vitro,* and/or have a significant inhibitory activity against *Mycobacterium smegmatis,* and have a low cytotoxicity *in vitro* on mammalian cells.

In addition, compounds **9** and **16** were further tested for their *in vitro* activity on several *Mycobacterium tuberculosis* strains, including drug resistant strains, on macrophages infected by *Mycobacterium tuberculosis* H37Rv, and compound **9** for its *in vivo* activity on mice infected by aerosol with *Mycobacterium tuberculosis* H37Rv. All the results show that this compound has a high inhibitory activity on the growth of *Mycobacterium tuberculosis in vitro* and *in vivo,* with a low cytotoxicity on macrophages and pneumocytes cell lines, which are the host cellular types targets of the tuberculosis bacillus.

The potent and remarkable specificity of compounds **9** and **16** on mycobacteria, including MDR-TB strains, suggests a novel biological target that is restricted to the mycobacteria. Moreover, these compounds displayed low cytotoxicity on various mammalian cell lines. A quite attractive feature is that compounds **9** and **16** not only have a MIC₉₉ comparable to the first-line anti-TB drugs but also display no crossresistance with INH and RIF. As the MIC measured on *M. tuberculosis* is below 6.25 µg/ml and its Selectivity Index is above 10, our results indicate that compounds **9** and **16** meet the criteria required for further tuberculosis drug development.

## Claims

1. Compound of the general formula : 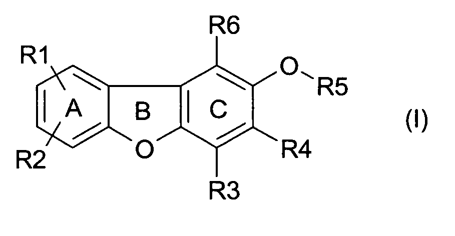 wherein :
- R1, R2 and R3 independently represent an hydrogen atom, a halogen atom , in particular F, Cl, or Br, a C₁-C₆ alkyl, in particular methyl or ethyl, CF₃, NO₂, NH₂, NR7, NHCOR8, OH, OR8, CN, O(C₁-C₆ alkyl)NR7, (C₁-C₆ alkyl)NR7, or NR8(C₁-C₆ alkyl)NR7, wherein NR7 represents a nitrogenous heterocycle, in particular 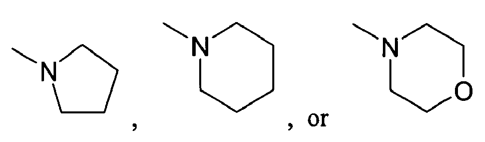 and R8 represents an hydrogen atom, or a C1-C6 alkyl.
- R4 and R6 represent an hydrogen atom, a halogen atom , in particular F, Cl, or Br, a C₁-C₆ alkyl, in particular methyl or ethyl, CF₃, NO₂, NH₂, NR7, NHCOR8, OH, OR8, CN, O(C₁-C₆ alkyl)NR7, (C₁-C₆ alkyl)NR7, or NR8(C₁-C₆ alkyl)NR7, wherein R7 and R8 are as defined above,
and R5 represents 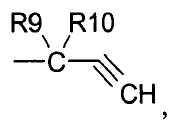
wherein R9 and R10 independently represent an hydrogen atom or a C₁-C₆ alkyl, in particular methyl or ethyl.
- or R4 represents an hydrogen atom, a halogen atom , in particular F, Cl, or Br, a C₁-C₆ alkyl, in particular methyl or ethyl, CF₃, NO₂, NH₂, NR7, NHCOR8, OH, OR8, CN, O(C₁-C₆ alkyl)NR7, (C₁-C₆ alkyl)NR7, or NR8(C₁-C₆ alkyl)NR7, wherein R7 and R8 are as defined above,
and R5 and R6 taken together form a pyran ring D1, according to the following formula : 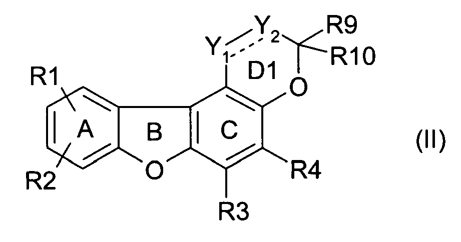
wherein R9 and R10 are as defined above, and wherein 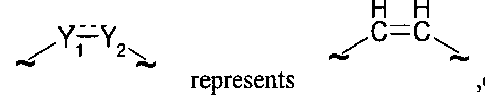 or 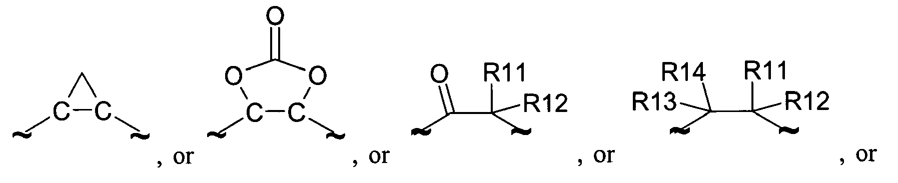 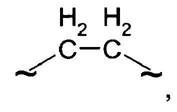 wherein R11, R12, R13 and R14 independently of each other represent an hydrogen atom, OH, O-C-O-(C₁-C₆)alkyl, or a (C₁-C₆)alkylcarbonyl(C₁-C₆)alkyl,
or R5 and R6 taken together form a oxacyclopentan ring D2 according to the following formula: 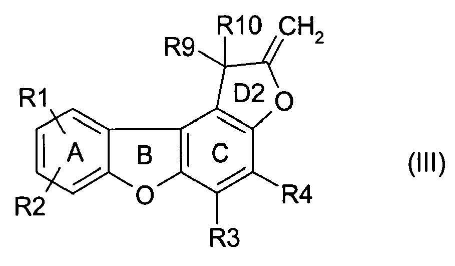 wherein R9 an R10 are as defined above,
- or R6 represents an hydrogen atom, a halogen atom , in particular F, Cl, or Br, a C₁-C₆ alkyl, in particular methyl or ethyl, CF₃, NO₂, NH₂, NR7, NHCOR8, OH, OR8, CN, O(C₁-C₆ alkyl)NR7, (C₁-C₆ alkyl)NR7, or NR8(C₁-C₆ alkyl)NR7, wherein R7 and R8 are as defined above, and R4 and R5 taken together form a pyran ring D3, according to the following formula : 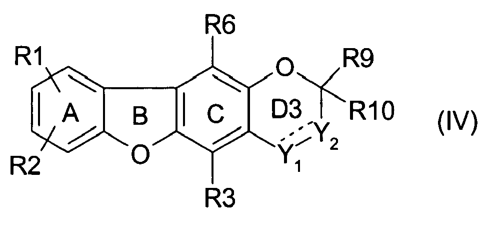
wherein R9, R10 and 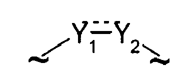 are as defined above,
or a pharmaceutically acceptable salt, an isomer, an enantiomer, or a diastereoisomer thereof, or a mixture thereof.

2. Compound according to claim 1, having the following general formula: 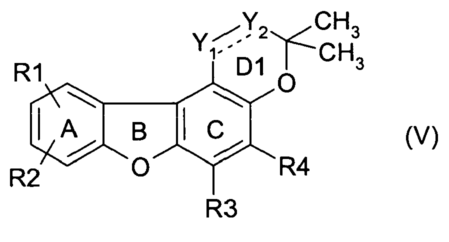 wherein R1, R2, R3, R4, and 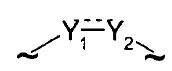 are defined according to claim 1,
or a pharmaceutically acceptable salt, an isomer, an enantiomer, or a diastereoisomer thereof, or a mixture thereof.

3. Compound according to claim 1, having the following general formula: 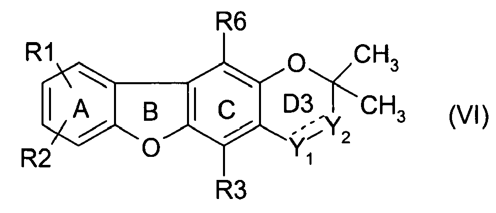 wherein R1, R2, R3, R6, and 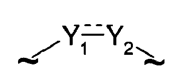 are defined according to claim 1,
or a pharmaceutically acceptable salt, an isomer, an enantiomer, or a diastereoisomer thereof, or a mixture thereof.

4. Compound according to anyone of claims 2-3, wherein 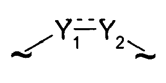 is 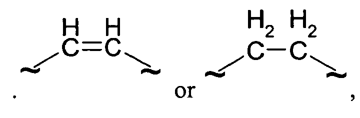 or a pharmaceutically acceptable salt, an isomer, an enantiomer, or a diastereoisomer thereof, or a mixture thereof.

5. Compound according to claim 1, having one of the following formulas: 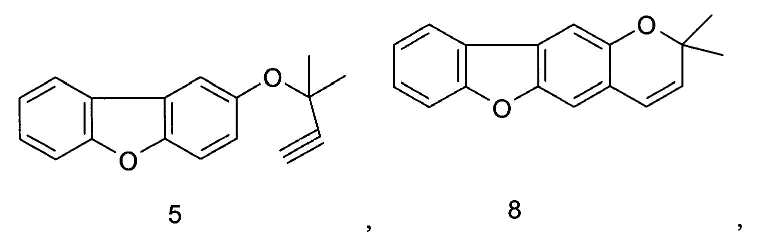 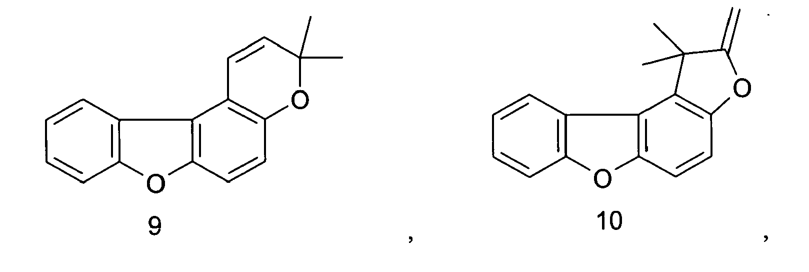 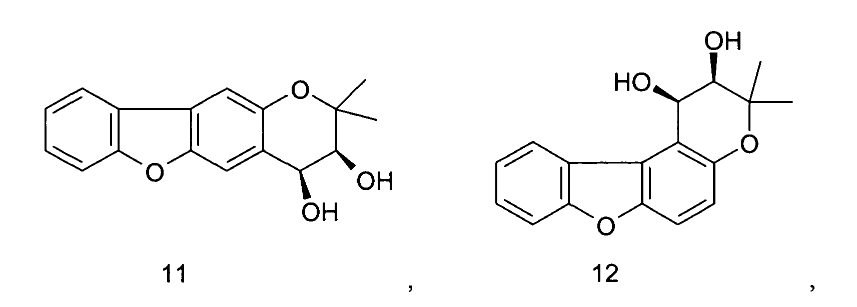 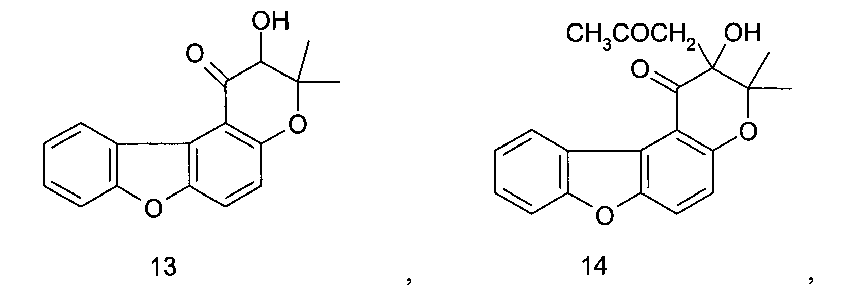 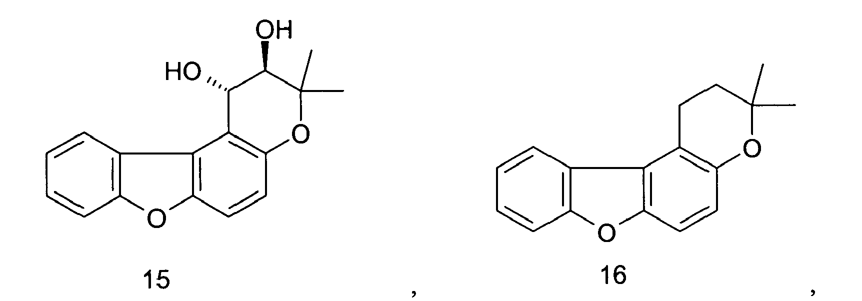 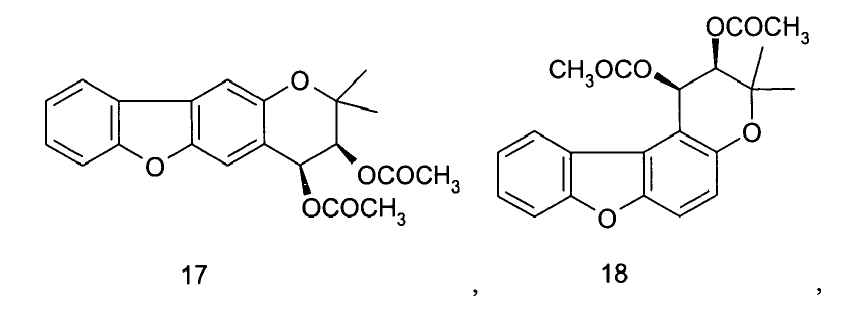 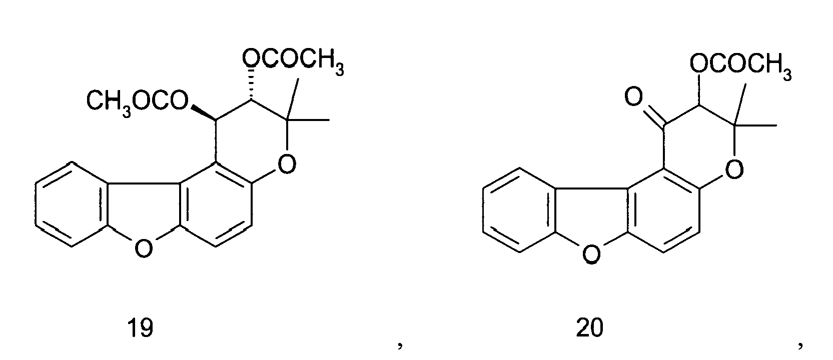 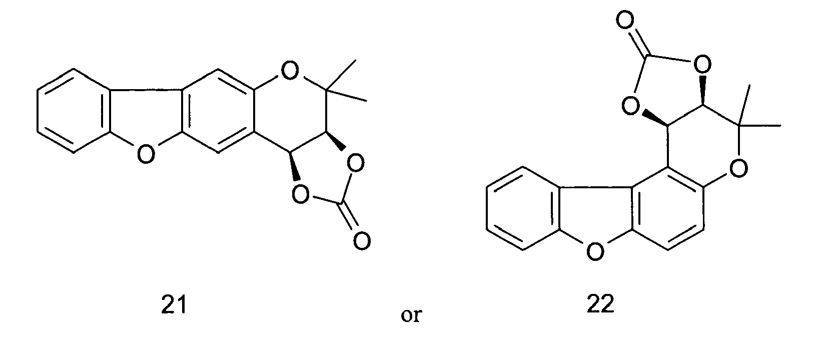 or a pharmaceutically acceptable salt, an isomer, an enantiomer, or a diastereoisomer thereof, or a mixture thereof.

6. Compound according to claim 5, having one of the following formulas: 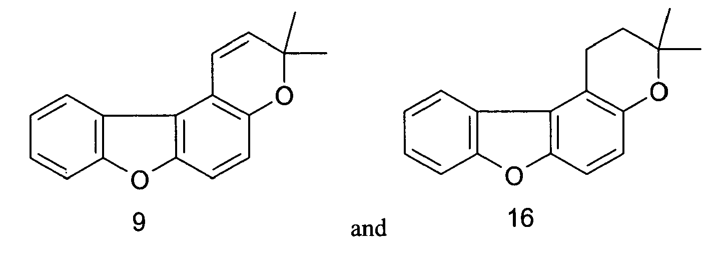 or a pharmaceutically acceptable salt thereof.

7. Method for preparing a compound having the following general formula : 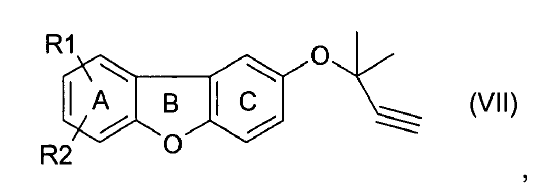 wherein R1 and R2 are as defined in claim 1, comprising:
(a) adding 1,8-Diazabicyclo[5.4.0]undec-7-ene (DBU) and copper chloride dihydrate to a cooled solution in anhydrous acetonitrile under inert atmosphere of a compound having the following formula 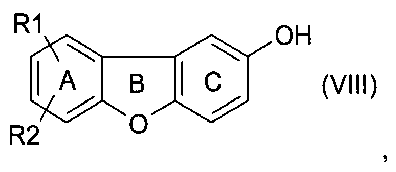 and then adding chlorobutyne,
(b) stirring, advantageously for 5 hours at 0°C,
(c) concentrating the mixture at reduced pressure
(d) partitioning the organic fraction and the aqueous fraction,
(e) washing the organic fraction successively with hydrochloric acid, sodium hydroxide, sodium hydrogenocarbonate and brine,
(f) removing the solvent to give compound of formula (VII).

8. Method for preparing a compound having the following general formula : 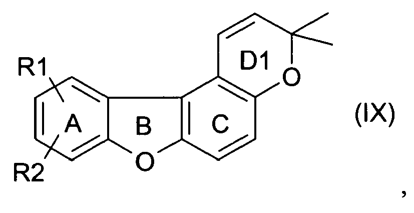 wherein R1 and R2 are as defined in claim 1, comprising:
(g1) heating the compound of formula 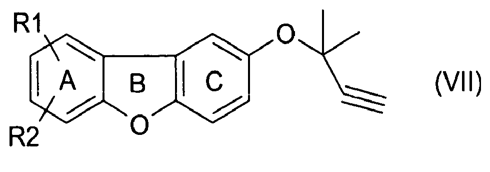 in boiling solvent, advantageously chosen from DMF, N,N-dimethylaniline, N,N-diéthylaniline and 1,3-dichlorobenzene, advantageously during 12 hours, and
(h1) purifying compound of formula (IX) by chromatography over silica gel and
(i1) recrystallizing the compound of formula (IX) in methanol.

9. Method for preparing a compound having the following general formula : 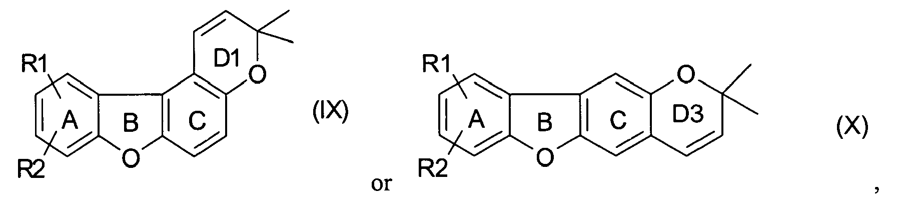 wherein R1, and R2 are as defined in claim 1, comprising:
(g2) suspending sodium hydride in dry solvent, advantageously chosen from toluene, benzene or xylene, under an inert atmosphere, and adding a compound of formula 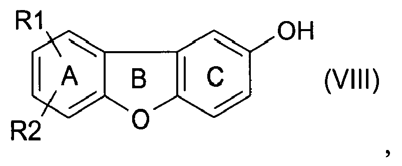
(h2) stirring at room temperature, advantageously for 30 minutes,
(i2) adding chlorobutyne and potassium iodide and refluxing the suspension, advantageously for three days,
(j2) removing the solvent, and purifying the residue by chromatography over silica gel in order to obtain a mixture of the compound of formula (IX) and of the compound of formula (X),
(k2) recrystallizing the mixture obtained in (j2) in methanol to obtain compound of formula (IX), or
(k3) purification of the compound of formula (X) by reverse phase HPLC.

10. Method according to any of claims 7-9, wherein R1 and R2 represent an hydrogen atom.

11. Pharmaceutical composition comprising at least one compound according to any of claims 1-6, with an acceptable carrier.

12. Pharmaceutical composition according to claim 11, further comprising at least one other antibiotic agent.

13. Compound according to any of claims 1-6 or pharmaceutical composition according to any of claims 11-12, as a medicament.

14. Compound or pharmaceutical composition according to claim 13, wherein said medicament is directed to the treatment of fungal or bacterial infections.

15. Compound or pharmaceutical composition according to claim 14, wherein said bacterial infection is a mycobacterial infection, including *Mycobacterium tuberculosis, Mycobacterium avium* and *Mycobacterium leprae* infections.

16. Compound or pharmaceutical composition according to claim 15, wherein said mycobacterial infection is caused by a *Mycobacterium tuberculosis* strain.

17. Compound or pharmaceutical composition according to claim 16, wherein said *Mycobacterium tuberculosis* strain is known to be resistant to at least one antibiotic agent.

18. Compound or pharmaceutical composition according to claim 14, wherein said bacterial infection is an infection by a bacterial strain that shares similarities in its cell wall composition with mycobacteria, in particular *Nocardia asteroides.*

19. Compound or pharmaceutical composition according to any of claims 13-18, wherein said medicament is used in combination with at least one other antibiotic agent.

20. Compound or pharmaceutical composition according to any of claims 13-19, wherein said medicament is directed to the treatment of immunodeficient patients infected by a Mycobacterial pathogenic strain.
